(19) Europäisches Patentamt
European Patent Office
Office européen des brevets

(11) Veröffentlichungsnummer: **0 115 613 B1**

(12) **EUROPÄISCHE PATENTSCHRIFT**

(45) Veröffentlichungstag der Patentschrift: **27.03.91**

(51) Int. Cl.5: **C12N 15/00**, C12P 21/02, C07H 21/04

(21) Anmeldenummer: **83112812.9**

(22) Anmeldetag: **20.12.83**

# (54) DNA-Sequenzen, deren Herstellung, diese Sequenzen enthaltende Plasmide und deren Verwendung zur Synthese eukaryotischer Genprodukte in Prokaryoten.

(30) Priorität: **24.12.82 DE 3247922**

(43) Veröffentlichungstag der Anmeldung:
**15.08.84 Patentblatt 84/33**

(45) Bekanntmachung des Hinweises auf die Patenterteilung:
**27.03.91 Patentblatt 91/13**

(84) Benannte Vertragsstaaten:
**AT BE CH DE FR GB IT LI LU NL SE**

(56) Entgegenhaltungen:
**EP-A- 0 042 246**
**EP-A- 0 047 600**
**EP-A- 0 062 971**
**EP-A- 0 095 702**
**DE-A- 3 247 923**

**NATURE, Band 276, 14. Dezember 1978, Seiten 684-689, Macmillan Journals Ltd.; G.F. MIOZZARI et al.: "The regulatory region of the TRP operon of Serratia Marcescens"**

(73) Patentinhaber: **BOEHRINGER INGELHEIM INTERNATIONAL GmbH**
**Postfach 20**
**W-6507 Ingelheim am Rhein(DE)**

(72) Erfinder: **Dworking, Marc-Bruce, Dr.**
**90 Morning-Side Drive Abt. 6/j**
**New York, NY 10027(US)**
Erfinder: **Dworkin-Rastl, Eva, Dr.**
**90 Morning Side Drive Abt. 6/j.**
**New Jork, NY 10027(US)**
Erfinder: **Adolf, Günther, Dr.**
**Johannagasse 20/7**
**A-1120 Wien(AT)**
Erfinder: **Hauel, Norbert, Dr. Dipl.-Chem.**
**Händelstrasse 12**
**W-7950 Biberach 1(DE)**
Erfinder: **Meindl, Peter, Dr.**
**Hockegasse 63/1**
**A-1120 Wien(AT)**
Erfinder: **Swetly, Peter, Dr.**
**Hietzinger Hauptstrasse 40 B/9**
**A-1130 Wien(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 115 613 B1

Rank Xerox (UK) Business Services

PROC. NATL. ACAD. SCI. USA, Band 78, Nr. 9, September 1981, Seiten 5543-5548; G. JAY et al.: "Construction of a general vector for efficient expression of mammalian proteins in bacteria: Use of a synthetic ribosome binding site"

CELL, Band 20, Juni 1980, Seiten 529-542, MIT; P.A. MEACOCK et al.: "Partitioning of bacterial plasmids during cell division: a Cis-acting locus that accomplishes stable plasmid inheritance"

GENE, Band 21, 1983, Seiten 237-248, Elsevier Biomedical Press; E. DWORKIN-RASTL et al.: Construction of expression plasmids producing high levels of human leukocyte-type interferon in Escherichia coli"

GENE, 21 (1983), S. 237-248

NATURE, 290 (1981), S. 20-26

Erfinder: Hauptmann, Rudolf, Dr.
Viktorgasse 25/8
A-1040 Wien(AT)

## Beschreibung

Eine Voraussetzung für die Expression von Genen in Bakterien ist das Vorhandensein eines sogn. Promotors, einer Erkennungssequenz für die Bindung der bakteriellen RNA-Polymerase. Ein Promotor ermöglicht also die Transkription der stromabwärts gelegenen Sequenzen. Gene, deren Produkte jederzeit synthetisiert werden, haben Promotoren, die immer zur Bindung von RNA-Polymerase-Molekülen fähig sind. Andere Gene bzw. Operons der Bakterien sind reguliert, d.h. ihr Promotor kann durch bestimmte Mechanismen zugänglich oder unzugänglich gemacht werden.

Eine weitere Voraussetzung für die Synthese der gewünschten Genprodukte ist eine effiziente Translation der RNA-Transkripte an den bakteriellen Ribosomen. So wurde von Shine und Dalgarno (siehe Nature 254, 34-38 (1975)) gezeigt, daß in Bakterien die Nukleotidsequenz am 5'-Ende der mRNA für deren Bindung ans Ribosom verantwortlich ist.

Das Ziel der vorliegenden Anmeldung ist es daher mit Hilfe eines bekannten Promotors, welcher über eine neue Ribosomenbindungs-/Linkersequenz mit einem Strukturgen verbunden ist, die gentechnologische Produktion von bakterienfremden Proteinen zu verbessern.

Gegenstand der vorliegenden Erfindung ist somit eine DNA-Sequenz der Formel

```
5'AATTCACGCTGATCGCTAAAACATTGTGCAAAAAGAGGGTTGACTTTGCCTTC
3'     GTGCGACTAGCGATTTTGTAACACGTTTTTCTCCCAACTGAAACGGAAG
                 |<-----------Promotor---------------------

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
---Promotor/Operator-------------------><--RBS--------->
                                                   Hin dIII
```

, an welche gegebenenfalls anschließend an die Ribosomenbindungssequenz eine Linkersequenz und die Sequenz eines Strukturgens für ein beliebiges Polypeptid, vorzugsweise für ein $\alpha$-Interferon, in derartiger Form angefügt ist, daß dieses durch Bakterien zur Expression gebracht wird und die Kombination der DNA-Sequenzen derartig erfolgt, daß die DNA-Sequenz des Strukturgens an die DNA-Linkersequenz durch 3',5'-Phosphodiesterbindung gekoppelt ist.

Diese Sequenz stellt eine neue Kombination aus einer literaturbekannten Promotorsequenz und einer literaturbekannten Ribosomenbindungssequenz dar.

Ein bevorzugter Gegenstand der vorliegenden Anmeldung ist jedoch derjenige, in welchem das Strukturgen mit der im PstI-Insert des Klons 1F7 (DM5-Nr. 2362) enthaltenen DNA-Sequenz, welche nur für ein reifes Interferon codiert, identisch ist, und welche die Formel

```
5'AATTCACGCTGATCGCTAAAACATTGTGCAAAAAGAGGGTTGACTTTGCCTTC
3'     GTGCGACTAGCGATTTTGTAACACGTTTTTCTCCCAACTGAAACGGAAG
                 |<-----------Promotor---------------------
```

```
GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
——Promotor/Operator——————————————>|<——RBS——————————>|
                                                  Hin dIII
```

```
        |---im PstI Insert des Klons 1F7 (DSM-Nr. 2362) enthal-
        tene für ein reifes IFN-α codierende DNA-Sequenz--->
TAAAGATGTGTGATCTGCCTCAAA
ATTTCTACACACTAGACGGAGTTT
|<---Linker---->|
```

aufweist.

Ein weiterer Gegenstand der vorliegenden Erfindung ist eine DNA-Ribosomenbindungs-/Linkersequenz der Formel

```
5'   TAAGGAGGTTTAAGCTTAAAGATGTGT
3'   ATTCCTCCAAATTCGAATTTCTACACACTAG     ,
                                   Sau3A
```

eine DNA-Linkersequenz der Formel

```
5'   AGCTTAAAGATGTGT
3'       ATTTCTACACACTAG
```

sowie eine DNA-Sequenz, welche aus der vorstehenden Linkersequenz und der im PstI-Insert des Klons 1F7 (DSM-Nr. 2362) vorhandenen für ein reifes IFN-α codierenden DNA-Sequenz ohne das $NH_2$-terminale Cystein dieses Interferons besteht, wobei beide Teile über die Sau3A-Enden so ligiert sind, daß das gesamte reife IFN-α codiert wird,

die die vorstehend erwähnten Sequenzen enthaltende Plasmide, z.B. das Plasmid pER103, welches eine DNA-Sequenz der Formel

```
5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
                |<————————Promotor———————————————————

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
——Promotor/Operator——————————————>|<——RBS——————————>|
                                              HindIII
```

enthält, und Plasmide, in denen anschließend an die einzige HindIII-Spaltstelle des Plasmids pER103 eine

Linkersequenz und die Sequenz eines Strukturgens für ein beliebiges Polypeptid, vorzugsweise für ein α-Interferon, in derartiger Form angefügt ist, daß dieses durch Bakterien zur Expression gebracht wird, z.B. die Plasmide pER33, pER21/1 und parpER33, wobei das Plasmid parpER33 zusätzlich einen par-Lokus enthält, und die Verwendung der so hergestellten Produktionsplasmide zur Herstellung eines beliebigen Polypeptids in Bakterien, insbesondere zur Herstellung von einem reifen α-Interferon wie dem durch den Klon 1F7 (DM5-Nummer 2362) codierten reifen α-Interferon, und Verfahren zu ihrer Herstellung.

Zur Erreichung des erfindungsgemäßen Zieles wird beispielsweise wie folgt verfahren:

Auswahl einer geeigneten bakteriellen Promotorsequenz:

Hierzu wird vorzugsweise ein Promotor verwendet, der, in Kombination mit einer Operatorsequenz, induzierbar bzw. reprimierbar ist. Ein derartiger Promotor hat den Vorteil, daß die Synthese des gewünschten bakterienfremden Proteins erst in einer späten Phase des bakteriellen Wachstumszyklus angeschaltet werden kann, z.B. im Falle des Tryptophanoperons (siehe Hallewell und Emtage in Gene 9 , 27-47 (1980)) durch Entzug von Tryptophan aus dem Kulturmedium und durch Zugabe von Indol-(3)-acrylsäure als Induktor des Tryptophanoperons zum Kulturmedium, und somit die Vermehrung der Bakterien nicht beeinflußt. Erfindungsgemäß wird zur Konstruktion des Plasmids pER103 das sehr starke und regulierbare literaturbekannte Promotor/Operator-System des Tryptophanoperons von Serratia marcescens (siehe Miozzari und Yanofsky in Nature 276, 684-689 (1978)) der Formel

```
5'AATTCACGCTGATCGCTAAAACATTGTGCAAAAAGAGGGTTGACTTTGCCTTC
3'    GTGCGACTAGCGATTTTGTAACACGTTTTTCTCCCAACTGAAACGGAAG
              |<---------Promotor---------------------

GCGAACCAGTTAACTAGTACACAAGTTCACGGCAACGG
CGCTTGGTCAATTGATCATGTGTTCAAGTGCCGTTGCC
---Promotor/Operator----------------->|
```

verwendet.

Konstruktion der Ribosomenbindungssequenz:

Hierzu wird die als besonders wirksam beschriebene Sequenz der Formel

5'TAAGGAGGTTTA

(siehe von Jay et al. in Proc. Nat. Acad. Sci. USA 78 , 5543-5548 (1981)) verwendet.

Die Konstruktion der Ribosomenbindungssequenz der Formel

```
       12mer
    ------------
5'  TAAGGAGGTTTA
3'  ATTCCTCCAAATTCGA
    ------ ----------
     6mer    10mer
```

erfolgt vorzugsweise durch Zusammenbau der 3 synthetischen Oligonucleotiden der Formeln

6mer

5' TCCTTA,

10mer

5' AGCTTAAACC und

12mer

5' TAAGGAGGTTTA

nach literaturbekannten Methoden, wobei vorher zweckmäßigerweise das 6mer Oligonucleotid radioaktiv markiert wird.

Die anschließende Verknüpfung des Promotor/Operators mit der Ribosomenbindungssequenz erfolgt nach literaturbekannten Verfahren.

Konstruktion des Plasmids pER103:

Aus einem Plasmid, z.B. dem Plasmid pBP101, das einen Teil des Tryptophanoperons von Serratia marcescens enthält, wird die 90 bp lange Promotor-/ Operator-Sequenz mit Hilfe der Restriktionsenzyme EcoRI und HaeIII herausgeschnitten. Dieses Fragment wird hierauf mit der synthetisch hergestellten RBS mit Hilfe des Enzymes DNA-Ligase verknüpft, und das so hergestellte Promotor-/ Operator-RBS-Fragment, das ein EcoRI-Ende vor dem Promotor/Operator und ein HindIII-Ende nach der RBS enthält, wird in das Plasmid pBR322 eingesetzt, anstelle des plasmideigenen 29 bp langen EcoRI-HindIII-Fragmentes. Die Wirksamkeit des so konstruierten Plasmids pER103 im Hinblick auf Expression von in die HindIII-Spaltstelle eingebauten Genen wird am Beispiel von einem $\alpha$-Interferon vom Subtyp A gezeigt.

Expression von einem $\alpha$-Interferon in pER103:

Interferon wird in menschlichen Zellen, wie andere zum Export bestimmten Proteine auch, als Präprotein synthetisiert, d.h. als Protein mit einer Leadersequenz. Diese Leadersequenz wird erst beim Austritt des Proteinmoleküles aus der Zelle von einem darauf spezialisierten Enzym abgespalten, wodurch die reife Form des Proteins erzeugt wird. Eine Möglichkeit, reifes Interferon von Bakterien synthetisieren zu lassen, ist eine Entfernung der Leadersequenz auf der DNA-Ebene, d.h. die Konstruktion eines Gens, das nur für die Sequenzen des reifen Proteins codiert.

Konstruktion von einem $\alpha$-Interferonproduktionsplasmid:

Beispielsweise hat Leukocyteninterferon eine Präsequenz von 23 Aminosäuren, darauf folgt ein Cystein, welches, nach Spaltung des Präinterferons, die erste Aminosäure des "reifen" Interferonpolypeptids darstellt. Zur Konstruktion von Plasmiden, die in Bakterien die Synthese von reifem Interferon ermöglichen sollten, ist es notwendig, ein Fragment des Interferongens zu isolieren, das exakt mit dem Codon für dieses Cystein beginnt. Vor dieses Cysteincodon muß dann ein ATG-Methionincodon gesetzt werden, um die Initiation der Proteinsynthese zu ermöglichen. Diese Konstruktion wird dann in geeigneter Weise, z.B. in das Expressionsplasmid pER103, eingesetzt, so daß der Abstand zwischen ATG-Initiationscodon und RBS für die Translation optimal ist. Ein $\alpha$-Interferon, das mit Hilfe eines solchen Plasmides in Bakterien hergestellt wird, besitzt die Aminosäuresequenz des reifen Polypeptids plus ein zusätzliches Methionin an dessen $NH_2$-terminalen Ende.

Beispielsweise wird das Plasmid pER33, das die durch den Klon 1F7 für ein reifes $\alpha$-Interferon codierende Sequenz enthält, erfindungsgemäß nach diesen Grundsätzen konstruiert (seine Herstellung ist in Fig. 4 schematisch wiedergegeben):

a) Konstruktion des für reifes Interferon codierenden Gens, mit Ausnahme des Codons für das $NH_2$-terminale Cystein:

Als Ausgangsmaterial für die Interferoninformation dient beispielsweise der für ein α-Interferon codierende cDNA-Klon 1F7, welcher am 17. Mai 1982 unter der DNS-Nummer 2362 bei der Hinterlegungsstelle "Deutsche Sammlung von Mikro-Organismen, Grisebachstraße 8, D 3400 Göttingen (Bundesrepublik Deutschland)" hinterlegt wurde. Er enthält das Interferongen insertiert in die PstI-Spaltstelle von pBR322. Dieses α-Interferon (wie auch andere Leukocyteninterferon-Subtypen) weist eine Spaltstelle für Sau3A gleich nach dem für das $NH_2$-terminale Cystein codierenden TGT auf. Die Existenz dieser Spaltstelle bildet die Grundlage für die verwendete Methode zur Konstruktion eines "reifen" Interferongens aus geeigneten Restriktionsfragmenten, z.B. aus dem interferonspezifischen 646 bp AvaII-Fragment und dem 34bp-Sau3A-AvaII-Fragment des Plasmids 1F7.

b) Herstellung des Oligonukleotid-Komplexes:

Zu dem so konstruierten Gen muß nun am 5'-Ende das fehlende Cysteincodon und, davor, ein ATG-Methionincodon hinzugefügt werden; weiters ist ein Verbindungsstück notwendig, das die Ligierung dieser Konstruktion in die HindIII-Spaltstelle des Expressionsplasmids pER103 ermöglicht. Zu diesem Zweck werden 4 Oligonukleotide synthetisiert: ein 14mer 5' TGTGATCTGCCTCA, ein 12mer 5' AGCTTAAAGATG, ein 9mer 5' CAGATCACA und ein 8mer 5' CATCTTTA. Diese Oligonukleotide sollen, nach Ligierung und einem Sau3A-Nachschnitt, das gewünschte Fragment darstellen, das den Interferongen-Teil (Sau3A-Ende) mit pER103 (HindIII-Ende) verbinden kann:

```
                      12mer          14mer
                  ┌────────────┐ ┌──────────────┐
          5'      AGCTTAAAGATGTGT:GATCTGCCTCA
                  3'  ATTTCTACACACTAGAC
                      └────────┘ └───────┘
   HindIII            8mer       9mer    :
                                         : Sau3A
                           MetCys
```

Erfindungsgemäß werden die Oligonukleotide so konstruiert, daß sich der Initiations-Codon ATG und der Cystein-Codon TGT auf separaten Fragmenten befinden. Dieses ermöglicht eine generelle Verwendung von 12mer (und 8mer) bei der Insertion von Genen in pER103. Das Oligonukleotid mit dem Cystein-Codon muß daher mindestens 9 Nukleotide lang sein. Beispielsweise können folgende Nukleotide verwendet werden:

5'TGTGATCTG,
5'TGTGATCTGC,
5'TGTGATCTGCC,
5'TGTGATCTGCCT oder
5 TGTGATCTGCCTC.

So wird beispielsweise das mit dem 14mer erhaltene ligierte Oligonukleotid mit Sau3A verdaut, um das ins Interferongen passende Sau3A-Ende zu erzeugen.

Die anschließende Verknüpfung von Interferongen und Oligonukleotid-Komplex, die Ligierung in ein Plasmid und dessen Transformation in einen bakteriellen Wirt, z.B. wie Escherichia coli HB 101, erfolgt nach literaturbekannten Verfahren.

Zur Erzielung einer weiteren Ausbeutesteigerung bei der Herstellung eukaryotischer Genprodukte kann es ferner von Vorteil sein, wenn das diesbezügliche Produktionsplasmid die für die Expression erforderlichen DNA-Sequenzen mehrfach, z.B. doppelt, enthält, beispielsweise zwei komplette Gene für ein reifes α-Interferon inklusive der bakteriellen, regulatorischen Sequenzen. Hierzu wird das mit einem bakteriellen Promotor, einer prokaryotischen, ribosomalen Bindungsstelle und eine ATG-Initiationscodon versehene Gen für reifes Interferon aus einem erfindungsgemäß hergestellten Produktionsplasmid, z.B. aus pER33, isoliert und nach Veränderung eines der beiden Enden dieses DNA-Stückes in ein mit einem Restriktionsenzym, z.B. EcoRI, erfindungsgemäß hergestellten linearsierten, vollständigen gleichen Plasmid eingefügt. Ferner ist es von Vorteil, wenn ein so erfindungsgemäß hergestelltes Tryptophanoperon tragendes Plasmid wie z.B. das Plasmid pER33 einen par-Lokus enthält, das heißt eine DNA-Sequenz, welche bei Abwesenheit eines Selektionsdruckes, z.B. durch ein Antibioticum wie Ampicillin, während des Bakterienwachstums für die gleichmäßige Weitergabe von Plasmiden in Tochterzellen verantwortlich ist (siehe P.M. Meacock, S.N. Cohen in Cell 20, 529-542 (1980)). Hierzu wurde der par-Lokus zunächst aus dem Plasmid pPM31, welches in der vorstehend genannten Literaturstelle beschrieben wird, isoliert und in ein erfindungsgemäß hergestelltes Interferonproduktionsplasmid eingebracht.

Durch die vorliegende Erfindung ist es somit gelungen, ein Expressionsplasmid zu konstruieren, das die Promotor-/Operator-Region des Tryptophan-Operons von Serratia marcescens, sowie eine synthetische RBS enthält. Die Wirksamkeit dieser Konstruktion zur gentechnologischen Produktion von bakterienfremden Proteinen wurde am Beispiel von einem α-Interferon gezeigt.

Die nachfolgenden Beispiele sollen die Erfindung näher erläutern:

## A. Beschreibung der allgemeinen Methoden:

### 1. Restriktionsenzymverdauungen

Restriktionsendonukleasen, beispielsweise der Firma Bethesda Research Laboratories, wurden unter folgenden Bedingungen verwendet:

EcoRI-, HindIII-, PstI- und AvaII-Verdauungen wurden in TA-Puffer durchgeführt (33 mM Tris-Acetat, pH 7,9, 66 mM K-Acetat, 10 mM Mg-Acetat, 100 µg/ml BSA); HaeIII-Verdauungen wurden in TM-Puffer durchgeführt (70 mM Tris-HCl, pH 7,5, 7 mM $MgCl_2$); Sau3A-Verdauungen wurden in 10 mM Tris-HCl, pH 7,4, 10 mM $MgCl_2$, 75 mM NaCl durchgeführt.

### 2. Plasmidpräparation, Gelelektrophorese

Plasmide wurden von 1,5 ml - oder von 100-300 ml - ÜbernachtKulturen in L-Broth + 25 µg/ml Tetracyclin oder + 100 µg/ml Ampicillin nach der Vorschrift von Birnboim und Doly (siehe Nucleic Acids Res. 7 , 1513-1523 (1979)) präpariert. Weitere Reinigung der Plasmide erfolgte durch Isopropanolfällungen (siehe unten) und (bei großen Ansätzen) durch Chromatographie an Sepharose 4B der Firma Pharmacia. Größere Mengen Plasmide (von 1-6 Liter Kultur) wurden nach der "cleared lysate"-Methode von Clewell und Helsinki (siehe Biochemistry 9 , 4428-4440 (1970)) aufgearbeitet, wobei anschließend daran eine Ethidiumbromid- CsCl-Gradientenzentrifugation durchgeführt wurde.

Elektrophorese von Plasmiden bzw. deren Restriktionsverdauungsprodukten erfolgte in 0,8 - 1,4 % Agarosegelen oder in 6 % Polyacryamidgelen, in 40 mM Tris-Acetat, pH 7,8, 20 mM Na-Acetat, 2 mM EDTA. Präparation von Restriktionsfragmenten erfolgte durch Ausschneiden des das gewünschte Fragment beinhaltenden Gelstückchens und elektrophoretische Elution der DNA aus dem Gel in einen Dialysenschlauch.

### 3. Kinase- und Phosphatasereaktionen, Ligasereaktionen

5'-Phosphorylierungsreaktionen (Endmarkierungen) wurden in TM-Puffer (70 mM Tris-HCl, pH 7,5, 7 mM $MgCl_2$) + 5 mM DTT + 0,2-0,5 mM ATP (10-20 µCi $^{32}$p-ATP) mit 5 Einheiten T4-Polynukleotidkinase, erhältlich von der Firma Bethesda Research Laboratories, 60 Minuten bei 37° C durchgeführt. Hierauf wurde 10 Minuten bei 70° C inkubiert, um das Enzym zu inaktivieren.

Ligasereaktionen wurden in TM-Puffer + 5 mM DTT + 0,25 mM ATP mit 0,1 Einheiten T4-DNA-Ligase, erhältlich von der Firma Bethesda Research Laboratories (bei "blunt-end"-Ligierungen), bzw. 0,005 Einheiten Ligase (Ligierungen von kohäsiven Enden) über Nacht bei 14° C durchgeführt. Anschließend wurde das Enzym 10 Minuten bei 70° C denaturiert.

Aufeinanderfolgende Kinase- und Ligasereaktionen wurden im selben Reaktionsgemisch durchgeführt. Nach Hitzedenaturierung des ersten Enzyms wurden wieder 5 mM DTT und 0,25-0,5 mM ATP zugegeben und die zweite Reaktion angeschlossen.

Phosphatasereaktionen wurden im Restriktionsverdauungspuffer (üblicherweise TA-Puffer) durchgeführt, durch Zugabe von 1 Einheit alkalischer Phosphatase (hergestellt von der Firma Sigma aus Kälberdarm) zu einer Restriktionsverdauung und Inkubation 15 Minuten bei 37° C. Daran wurden 1-2 Phenolextraktionen, eine Ätherextraktion und eine Alkoholfällung angeschlossen. Oft wurden hierauf die DNA-Fragmente noch elektrophoretisch aufgetrennt und geleluiert, bevor weitere Manipulationen durchgeführt wurden.

Um große DNA-Fragmente (500 bp und größer) von kleinen Fragmenten (Linkerfragmenten, die nicht ligiert hatten, oder kleinen Restriktionsverdauungsprodukten) abzutrennen, wurden Isopropanolfällungen durchgeführt: Die Reaktion wurde mit 2N $NH_4$-Acetat (Endkonzentration) versetzt und mit 0,6 Volumen Isopropanol 10-20 Minuten bei Raumtemperatur gefällt. Nach Zentrifugation 5 Minuten in einer Eppendorfzentrifuge wurde der Überstand entfernt, die Fällung mit kaltem 70 % Äthanol gewaschen, nochmals zentrifugiert; das daraus resultierende Pellet wurde getrocknet und war für weitere Manipulationen bereit.

B. Herstellung der Oligonukleotide:

Legende:

| | | |
|---|---|---|
| DMTr | = | p,p'-Dimethoxy-triphenylmethyl |
| ibu | = | Isobutyryl |
| bz | = | Benzoyl (am Basenstickstoff) |
| 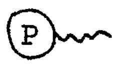 | = | polymeres Trägermaterial |
| B | = | Thymin oder<br>$N^2$-Isobutyrylguanin oder<br>$N^4$-Benzoylcytosin oder<br>$N^6$-Benzoyladenin |
| THF | = | Tetrahydrofuran |

Beispiel I

Funktionalisierung des polymeren Trägermaterials

Die Funktionalisierung des polymeren Trägermaterials erfolgte nach literaturbekannten Verfahren. Als Trägermaterial diente HPLC-Silicagel (Macherey 5 Nagel, Korngröße 20 µm, Porengröße 200 Å). Es wurde derivatisiert wie bei Mateucci und Caruthers beschrieben, mit der Ausnahme, daß der Succinylierungsschritt mit Bernsteinsäureanhydrid in wasserfreiem Pyridin durchgeführt wurde (siehe M.D. Matteucci, M.H. Caruthers, Tetrahedron Letters 21 , 719 (1981), J. Am. chem. Soc. 103, 3185 (1981) und auch T. Tanaka, R.L. Letsinger, Nucleic Acids Research 10 , 3249 (1982)). Die geschützten Nucleoside wurden so entsprechend folgendem Formelbild kovalent mit dem Silicalgel verknüpft:

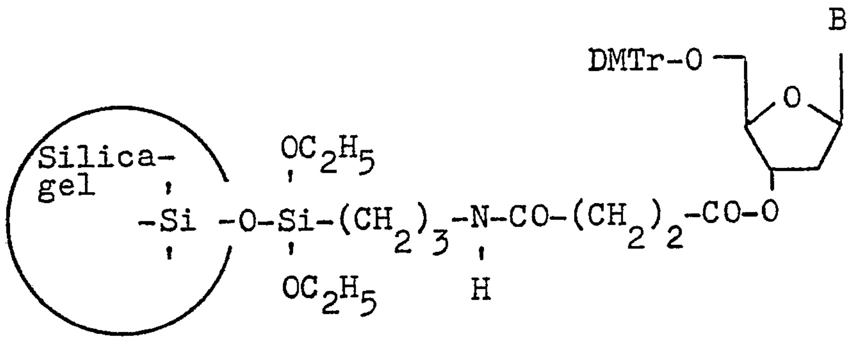

Die Belastungsdichte betrug zwischen 68 und 104 µMol Nucleosid pro g Trägermaterial.

Beispiel II 5,-Dimethoxytrityl-desoxythymidin-3'-chlormethoxyphosphit

Die voll geschützten Nucleosid-3'-chlormethoxyphosphite wurden nach literaturbekannten Verfahren synthetisiert (M.D. Matteucci, M.H. Caruthers, J. Am. Chem. 103 , 3185 (1981) und T. Tanaka, R.L. Letsinger, Nucleic Acids Research 10 , 3249 (1982)).

544,6 mg (1,0 mMol) 5'-Dimethoxytrityl-thymidin (DMTrdT) wurden in 1,0 ml absolutem THF gelöst und diese Lösung bei -78° C innerhalb 15 Minuten unter Argon tropfenweise zu einer gerührten Lösung von 0,9 mMol Methyl-dichlorphosphit in 0,5 ml absolutem Pyridin und 2,0 ml absolutem THF gegeben. Nach weiteren 10 Minuten wurde die Reaktionslösung auf Raumtemperatur erwärmt und zentrifugiert. Der Überstand wurde mit einer Pipette in einen trockenen, mit Argon gefüllten Schliffkolben (25 ml) überführt. Bei Raumtemperatur wurde unter Vakuum das Lösungsmittel abgezogen, danach je 0.5 ml Toluol und THF hinzugegeben und erneut eingedampft, wobei als Produkt ein farbloser schaumiger Feststoff zurückblieb. Dieses Produkt wurde nicht auf seine Reinheit hin analysiert, sondern in 10,0 ml absolutem Pyridin gelöst und diese Lösung unter Argon bei -20° C bis zur weiteren Verwendung, maximal jedoch eine Woche lang, aufbewahrt.

Beispiel III

5'-Dimethoxytrityl-$N^2$-isobutyryl-desoxyguanosin-3'-chlor methoxy-phosphit

Analog Beispiel II wurde eine Lösung dieses Nucleosidphosphoro-chloridits in 10,0 ml Pyridin aus 640,0 mg (1,0 mMol) 5'-Dimethoxytrityl-$N^2$-isobutyl-desoxyguanosin hergestellt.

Beispiel IV

5'-Dimethoxytrityl-$N^6$-benzoyl-desoxycytidin-3'-chlorme thoxy-phosphit

Analog Beispiel II wurde eine Lösung dieses Nucleosidphosphoro-chloridits in 10,0 ml Pyridin aus 633,7 mg (1,0 mMol) 5'-Dimethoxy-trityl-$N^4$-benzoyl-desoxycytidin hergestellt.

Beispiel V 5'-Dimethoxytrityl-$N^6$-benzoyl-desoxyadenosin-3'-chlorme thoxy-phosphit

Analog Beispiel II wurde eine Lösung dieses Nucleosidphos phoro-chloridits in 10,0 ml Pyridin aus 657,7 mg (1,0 mMol) 5'-Dimethoxytrityl-$N^6$-benzoyl-desoxyadenosin hergestellt.

Beispiel VI

Synthese von d-TCCTTA

50 mg (= 5 µMol) des mit DMTrdA$^{bz}$ beladenen polymeren Trägers (siehe Beispiel I) wurden in eine Glasfritte gefüllt. Entsprechend der folgenden Auflistung wurden dann verschiedene Lösungsmittel und Reagenzienlösungen hinzugefügt, der Träger darin kurz aufgeschüttelt, und die Lösung nach der gewünschten Umsetzung wieder entfernt, indem sie mit Hilfe eines Argon-Gasstromes von oben durch die Fritte hindurchgepreßt wurde.

a) Abspaltung der DMTr-Gruppe mit 3 ml einer Lösung aus 70 g Zinkbromid, 500 ml Nitromethan und 5 ml Wasser.
Reaktionszeit: 10 Minuten.
b) 4 x Waschen mit je 3 ml einer Mischung aus n-Butanol-Lutidin-THF (4:1:5).
c) 4 x Waschen mit je 4 ml absolutem Pyridin.
d) Ankondensation des nächsten Nucleotid-Bausteines:
1 ml der Pyridin-Lösung von 5'-Dimethoxytrityl-desoxythymidin-3'-chlormethoxyphosphit (Beispiel B) (ca. 100 µMol) wurden unter Argon in die Fritte zu dem polymeren Träger gegeben und dieser in der Lösung aufgeschüttelt.
Reaktionszeit: 10 Minuten.
e) 3 x Waschen mit je 3 ml absolutem Pyridin.
f) Oxidation des Phosphorigsäure-triesters mit 100 mg Jod, gelöst in 3 ml eines Gemisches aus THF, Lutidin und Wasser (2:2:1).
Reaktionszeit: 7 Minuten.
g) 3 x Waschen mit je 4 ml THF.
h) Acetylierung der nicht umgesetzten 5'-OH-Gruppen mit einer Lösung aus 150 mg 4-Dimethylaminopyridin, 0,3 ml Collidin, 0,25 ml Acetanhydrid und 2,5 ml THF.
Reaktionszeit: 5 Minuten.
i) 4 x Waschen mit je 3 ml Nitromethan. Dieser Zyklus von a) bis i) wurde nun vier weitere Male wiederholt, wobei bei Schritt d) der für die Sequenz jeweils benötigte NucleotidBaustein eingesetzt wurde.

Ausbeute-Bestimmung:

Nach Ankondensieren des letzten Nucleotid-Bausteines wurde das Trägermaterial im Ölpumpenvakuum getrocknet, eine Probe von ca. 1 mg genau abgewogen und mit 10,0 ml einer 0,1 M Lösung von Toluolsulfonsäure in Acetonitril versetzt. Durch die dabei eintretende Abspaltung des Dimethoxytrityl-Kations erhält man eine orange-rot gefärbte Lösung, deren Absorption bei 498 nm gemessen wurde. Nach der Formel

$$\text{Beladung } [\mu\text{Mol/g}] = \frac{(\text{Abs.}^{498}) \times (\text{Verdünnungsfaktor}) \times 14{,}3}{\text{Gewicht des Trägers [mg]}}$$

läßt sich die Beladung des Träger-Materials mit Dimethoxytrityl-Schutzgruppen berechnen. Man erhielt: 43 µMol/g. Dies entspricht einer durchschnittlichen Ausbeute von 85 % pro Kondensationsschritt.

Abspaltung der Methylreste von den Phosphorsäuretriester-Gruppen:

Das Trägermaterial wurde 45 Minuten lang in 4 ml einer Lösung von Thiophenol, Triethylamin und Dioxan (1:1:2) geschüttelt, anschließend mit Methanol, dann mit Ether gewaschen.

Abspaltung der Basen-Schutzgruppen und gleichzeitige Abspaltung der Hexanucleotid-Kette vom polymeren Träger:

Das Trägermaterial wurde 14 Stunden lang mit 10 ml konz. Ammoniak auf 50 °C erwärmt, die wäßrige Lösung dann abgesaugt und das Filtrat auf ca. 2 ml eingeengt.

Reinigung des Produkts:

Das so erhaltene Rohprodukt, das am 5'-Ende noch eine Dimethoxytrityl-Schutzgruppe trug, wurde der Reversed-Phase HPLC unterworfen. Säule: μ-Bondapak $C_{18}$ Fa. Waters; Eluans: 0,1 M Triethylammoniumacetatpuffer pH 7 mit 25 % Acetonitril; Fluß 2 ml/min.; Retentionszeit: 14 Minuten. Die gesammelten Elutionsfraktionen wurden auf ca. 1 ml eingeengt, mit 10 ml 80 % Essigsäure versetzt und 30 Minuten bei Raumtemperatur stehengelasen. Dann wurde bei 50° C im Vakuum zur Trockne eingeengt, der Rückstand in 25 ml Wasser gelöst und das abgespaltene Dimethoxytritanol mit 3 x 15 ml Ether extrahiert. Die wäßrige Phase wurde erneut bis zur Trockne eingeengt, der Rückstand in 2,5 ml Wasser gelöst, über Biogel P 2 (Säule: 60 x 1,7 cm) entsalzt und lyophilisiert.

Analyse des Produkts:

Als Reinheitskontrolle diente das analytische HPLC-Diagramm (Säule: 300 x 3,9 mm, μ-Bondapak $C_{18}$, Fa. Waters; Eluans: 0,1 M Triethylammoniumacetatpuffer pH 7 mit 12 % Acetonitril; Fluß: 1,5 ml/min.; Retentionszeit: 3,7 Minuten).

Beispiel VII

Synthese von d-TAAGGAGGTTTA

Hergestellt analog Beispiel VI ausgehend von 300 mg (30 μMol)

$DMTrdA^{bz}$~(P) .

HPLC-Diagramm des Produkts:
Säule: 300 x 3,9 mm, μ-Bondapak $C_{18}$, Fa. Waters;
Eluans: 0,1 M Triethylammoniumacetatpuffer pH 7 mit 12 % Acetonitril; Fluß: 1,5 ml/min; Retentionszeit: 4,4 Minuten

Beispiel VIII

Synthese von d-AGCTTAAACC

Hergestellt analog Beispiel VI ausgehend von 200 mg (16 μMol)

$DMTrdC^{bz}$~(P) .

HPLC-Diagramm des Produkts:
Säule: 300 x 3,9 mm, μ-Bondapak $C_{18}$, Fa. Waters;
Eluans: 0,1 M Triethylammoniumacetatpuffer pH 7 mit 12 % Acetonitril; Fluß: 1,5 ml/min; Retentionszeit: 3,4 Minuten.

Beispiel IX

Synthese von d-CATCTTTA

Hergestellt analog Beispiel VI ausgehend von 150 mg

(1,32 µMol)

$DMTrdA^{bz}$ ~Ⓟ .

HPLC-Diagramm des Produkts:
Säule: 300 x 7,8 mm, µ-Bondapak $C_{18}$, Fa. Waters;
Eluans: 0,1 M Triethylammoniumacetatpuffer pH 7 mit 20 % Acetonitril; Fluß: 1,5 ml/min; Retentionszeit: 7,7 Minuten.

Beispiel X

Synthese von d-AGCTTAAAGATG

Hergestellt analog Beispiel VI ausgehend von 200 mg (16,2 µMol)

$DMTrdG^{ibu}$ ~Ⓟ .

HPLC-Diagramm des Produkts:
Säule: 300 x 7,8 mm, µ-Bondapak $C_{18}$, Fa. Waters;
Eluans: 0,1 M Triethylammoniumacetatpuffer pH 7 mit 26 % Acetonitril; Fluß: 1,5 ml/min; Retentionszeit: 5,2 Minuten.

Beispiel XI

Synthese von d-TGTGATCTGCCTCA

Hergestellt analog Beispiel VI ausgehend von 250 mg (22 µMol)

$DMTrdA^{bz}$ ~Ⓟ .

HPLC-Diagramm des Produkts:
Säule: 300 x 7,8 mm; µ-Bondapak $C_{18}$; Fa. Waters;
Eluans: 0,1 M Triethylammoniumacetatpuffer pH 7 mit 25 % Acetonitril; Fluß: 1,5 ml/min; Retentionszeit: 6,1 Minuten.

Beispiel XII

Synthese von d-CAGATCACA

Hergestellt analog Beispiel VI ausgehend von 150 mg (13,2 µMol)

$DMTrdA^{bz}$ ~Ⓟ .

HPLC-Diagramm des Produkts:
Säule: 300 x 7,8 mm; µ-Bondapak $C_{18}$; Fa. Waters;

Eluans: 0,1 M Triethylammoniumacetatpuffer pH 7 mit 20 % Acetonitril; Fluß: 0,2 ml/min; Retentionszeit: 5,2 Minuten.

Analyse der Oliqodeoxynucleotide

Die Sequenzanalyse der synthetisch hergestellten Oligodeoxynucleotide wurde durchgeführt, nachdem diese in das Interferonproduktionsplasmid pER33 eingebaut worden waren (siehe Beispiel 2: Sequenzanalyse des Interferonplasmids pER33). Mit dieser Analyse wird gleichzeitig die Richtigkeit der Basenabfolge in den Oligooxynucleotiden belegt (siehe Fig. 5).

Beispiel 1

Konstruktion des Plasmids pER103

Die Konstruktion des Plasmids pER103 ist schematisch in Fig. 1 dargestellt.

a) Isolierung des Promotor/Operator-Fragmentes

Das Plasmid pBR101, das etwa 1000 bp des Tryptophan-Operons von Serratia marcescens enthält, stellte das Ausgangsmaterial für die Isolierung der Promotor/Operator-Region dar. Diese regulatorische Region liegt innerhalb eines 90 bp langen EcoRI - HaeIII-Fragmentes, in dem der Promotor in der Richtung EcoRI → HaeIII orientiert ist. Etwa 25 µg des Plasmides pBP101 wurden mit dem Restriktionsenzym EcoRI verdaut, die zwei entstehenden Fragmente wurden durch Gelelektrophorese (1,4 % Agarose) voneinander getrennt und das 200 bp lange Fragment wurde elektrophoretisch aus dem Gel eluiert. Dieses Fragment wurde dann mit HaeIII verdaut, die beiden Verdauungsprodukte wurden auf einem 6 % Polyacrylamidgel getrennt und das 90 bp lange Fragment (Promotor/Operator) wurde wieder aus dem Gel isoliert.

b) Konstruktion der Ribosomenbindungssequenz (RBS)

Die RBS wurde aus 3 synthetischen Oligonukleotiden zusammengesetzt: dem 6mer 5'-TCCTTA, dem 10mer 5'-AGCTTAAACC und dem 12mer 5'-TAAGGAGGTTTA. 500 pMole vom 6mer wurden mit Hilfe des Enzyms Polynukleotidkinase phosphoryliert und dabei radioaktiv markiert (siehe Teil A). Das Reaktionsgemisch wurde 10 Minuten lang auf 70 °C erhitzt, um die Kinase zu inaktivieren, hierauf wurden äquimolare Mengen von (nicht phosphoryliertem) 10mer und 12mer zugesetzt, das Oligonukleotidgemisch wurde auf 95 °C erhitzt und dann langsam (über etwa 3 Stunden) auf 30-35 °C abgekühlt, wobei die Oligonukelotide miteinander hybridisierten:

```
         12mer
     ┌──────────────┐
5'   │ dTAAGGAGGTTTA          3'
HO   │
3'     dATTCCTCCAAATTCGA      5'
       └─────┘.└───────┘
        6mer   10mer
```

Durch Zusatz von 0,25 mM ATP und 5mM DTT wurden 6mer und 10mer mit Hilfe des Enzyms DNA-Ligase kovalent miteinander verbunden (siehe Teil A). Das Fehlen von Phosphatresten an den 5'-Enden des 12mers und des 10mers verhinderte das Ent stehen von multimeren Ligationsprodukten. Nach der Reaktion wurde 10 Minuten auf 70 °C erhitzt, um die Ligase zu inaktivieren, hierauf wurden nach Zugabe von 0,5 mM ATP und 5 mM DTT in einer weiteren Kinasereaktion (siehe Teil A) auch die 5'-Enden des 12mers und des 10mers kinasiert, wodurch die RBS fertiggestellt war.

c) Verknüpfung von Promotor und RBS

12 pMole des 90 bp langen EcoRI-HaeIII Promotor-/ Operator-Fragmentes wurden unter Standardbedingungen (siehe Teil A) mit 60 pMolen RBS ligiert. Da nur das durch den HaeIII-Schnitt erzeugte stumpfe Ende ("blunt end") des 90 bp-Fragmentes mit dem stumpfen Ende der RBS ligieren kann,

entstanden nur Moleküle, die die RBS in der richtigen Orientierung stromabwärts vom Promotor enthielten. Nach der Reaktion wurde die Ligase 10 Minuten bei 70 °C inaktiviert, es wurde auf TA-Puffer-Konzentration (siehe Teil A) eingestellt und mit 200 Einheiten HindIII und 10 Einheiten EcoRI nachverdaut. Dieses war vorteilhaft, um die in der Ligasereaktion entstandenen Multimeren (da neben der gewünschten Reaktion sowohl EcoRI-Enden, als auch HindIII-Enden miteinander ligieren konnten) wieder in Monomere zu verwandeln. Hierauf wurde die Probe auf einem 6 % Polyacrylamidgel aufgetrennt und das etwa 100 bp lange promotor/Operator-RBS-Fragment (das ein EcoRI- und ein HindIII-Ende besitzt) aus dem Gel herausgeschnitten und elektrophoretisch eluiert, um es vom Überschuß der nicht ligierten RBS abzutrennen. Hiermit war der für die Expression verantwortliche Teil des Plasmids fertiggestellt (siehe Fig. 3).

Die in Fig. 3 dargestellte Nukleotidsequenz läßt sich durch literaturbekannte Methoden der Polynukleotidsynthese nachvollziehen.

d) Insertion des promotor/Operator-RBS-Fragments in das Plasmit pBR322 Etwa 2 µg des Plasmides pBR322 wurden mit den Restriktionsenzymen EcoRI und HindIII geschnitten, wobei zwei Fragmente entstanden: ein großes mit 4332 bp und ein kleines mit 29 bp. Das große Fragment wurde durch Elektrophorese auf einem 0,8 % Agarosegel vom kleinen Fragment getrennt, aus dem Gel herausgeschnitten und eluiert. Etwa 0,4 pMole dieses Fragments wurden dann mit etwa 10 pMolen des Promotor/Operator-RBS-Fragmentes ligiert (siehe Teil A). Das pBR322-Fragment konnte wegen seiner zwei nicht zusammenpassenden überhängenden Enden nicht mit sich selbst ligieren; da das Promotor/-Operator-RBS-Fragment nur in einer Orientierung in das Plasmid ligiert werden konnte, erfolgte Promotion in Richtung HindIII-Spaltstelle, zum Tetracyclinresistenz-Gen von pBR322.

e) Transformation von Escherichia coli mit dem Plasmid pER103

Escherichia coli HB 101 wurde mit dem Reaktionsgemisch dieser letzten Ligierung auf literaturbekannte Weise transformiert (siehe Dworkin und Dawid, Dev. Biol. <u>76</u> , 435-448 (1980)) und die Transformanten wurden auf ampicillinhaltigen Agarplatten selektioniert. Hierfür wurden E. coli HB 101 Zellen zu einer Dichte von ca. 2 x $10^8$ Zellen/ml aufgezüchtet. Die Zellen wurden pelletiert und in 100 mM $CaCl_2$-Lösung suspendiert (20 Minuten bei 0 °C). Danach wurden die Zellen mit dem Reaktionsgemisch der Ligasereaktion 5 Minuten bei 0 - 4 °C und 5 Minuten bei 37 °C inkubiert. Nach Zugabe von 0,5 - 1 ml 1-Broth wurde 15 - 30 Minuten bei 37 °C weiterinkubiert. 19 Transformanten wurden ausgewählt und mit Hilfe von HaeIII-Restriktionsverdauungen auf ihren möglichen Gehalt des Promotor/Operator-RBS-Fragmentes geprüft. Das 192 bp lange pBR322/HaeIII-Fragment fehlte und war durch ein 264 bp-Fragment ersetzt worden (16 bp von der HaeIII-Spaltstelle in pBR322 "links" von der EcoRI-Spaltstelle + 103 bp Promotor/ Operator-RBS-Fragment + 145 bp von der HindIII-Spaltstelle bis zur nächsten HaeIII-Spaltstelle "rechts" davon, in pBR322). Von den 19 ausgewählten Transformanten zeigten 18 das erwartete Verdauungsmuster (siehe Fig. 2).

f) Sequenzanalyse der Promotor/Operator-RBS-Region des Plasmids pER103

Um die Richtigkeit der konstruierten Plasmide festzustellen, wurde eines der Plasmide, welches als pER103 bezeichnet wurde, ausgewählt und in der Promotor/Operator-RBS-Region sequenziert und deren Position in pBR322 festgestellt. Die Sequenzanalyse wurde nach der literaturbekannten Methode von Maxam und Gilbert (siehe Proc. Nat. Acad. Sci. <u>74</u> , 560-564 (1977)) durchgeführt und erfolgte von der EcoRI-Spaltstelle in Richtung HindIII-Spaltstelle (und darüber hinaus), wie auch von der HindIII-Spaltstelle in Richtung EcoRI-Spaltstelle (und darüber hinaus). Es ergab sich die Nukleotid-Sequenz, welche in Fig. 3 dargestellt ist.

pER103 ist somit ein Plasmid, das die Promotor/Operator-Region des Tryptophanoperons von Serratia marcescens in Kombination mit einer synthetischen RBS enthält. Dieses neue Plasmid promoviert die Transkription von Genen, die in seine HindIII-Spaltstelle eingebaut werden, und ermöglicht eine effiziente Translation dieser Transkriptionsprodukte. Daß dieses der Fall ist, wird in Beispiel 2 gezeigt.

## <u>Beispiel 2</u>

### <u>Konstruktion des Plasmids pER33</u>

a. Konstruktion des für ein reifes α-Interferon codierenden Gens, mit Ausnahme des Codons für das $NH_2$-terminale Cystein

Etwa 1 µg des PstI-Insertes von 1F7 wurde mit dem Restriktionsenzym Sau3A verdaut, und das 177 bp lange Fragment, das von der Sau3A-Spaltstelle nach dem $NH_2$-terminalen Cysteincodon bis zur

nächsten Sau3A-Spaltstelle reicht und eine AvaII-Spaltstelle beinhaltet, wurde von einem 6 % Polyacryl amidgel isoliert. Es wurde mit 1 Einheit alkalischer Phosphatase behandelt (siehe Teil A) und nach Entfernung der Phosphatase durch Phenol- und Ätherextraktion mit Äthanol gefällt. Das Fragment wurde anschließend mit AvaII geschnitten, wodurch das gewünschte 34 bp Sau3A-AvaII-Fragment und ein 143 bp-Fragment erhalten wurden.

Parallel dazu wurde das interferonspezifische 646 bp AvaII-Fragment, das von der AvaII-Spaltstelle innerhalb des 177 bp Sau3A-Fragmentes bis hinter das Terminationscodon reicht, aus dem Plasmid 1F7 präpariert. Etwa 0,5 µg dieses 646 bp AvaII-Fragmentes wurden nun zusammen mit der Mischung aus 34 bp und 143 bp Sau3A-AvaII-Fragmenten mit dem Enzym DNA-Ligase inkubiert (Ligierung von kohäsiven Enden, siehe Teil A). Dadurch entstanden AvaII-Fragmente, die, kovalent verbunden, von AvaII-Sau3A-Fragmenten flankiert sind. Sobald ein AvaII-Sau3A-Fragment mit einem AvaII-Fragment ligiert hat, können an dieser Stelle keine weiteren Ligierungen mehr stattfinden, da die Sau3A-Enden dephosphoryliert worden waren. Nach der Ligasereaktion wurde 10 Minuten auf 70 °C erhitzt, um das Enzym zu inaktivieren, dann wurden 5 mM DTT und 0,25 mM ATP zugegeben, und die dephosphorylierten Sau3A-Enden wurden wieder kinasiert (siehe Teil A). Das Reaktionsgemisch wurde auf einem 6 % Polyacrylamidgel aufgetrennt und Moleküle im Bereich von 700-800 bp (ein 646 bp AvaII-Fragment flankiert von zwei AvaII-Sau3A-Fragmenten) und im Bereich von 1300 - 1500 bp (zwei miteinander ligiert 646 bp AvaII-Fragmente flankiert von AvaII-Sau3A-Fragmenten) wurden eluiert.

b. Präparation des Oligonukleotid-Komplexes der Formel

```
                  12mer          14mer
      5'   AGCTTAAAGATGTGTGATCTGCCTCA
                3'  ATTTCTACACACTAGAC

HindIII           8mer       9mer
                                   Sau3A
                      MetCys
```

4 synthetische hergestellte Oligonukleotide, nämlich ein 14mer 5' TGTGATCTGCCTCA, ein 12mer 5' AGCTTAAAGATG, ein 9mer 5' CAGATCACA und ein 8mer 5' CATCTTTA, wurden folgendermaßen umgesetzt: je 250 pMole 8mer, 9mer und 14mer wurden phosphoryliert (siehe Teil A), nach der Reaktion wurde die Kinase durch Erhitzen auf 95 °C inaktiviert, es wurden 250 pMole nicht kinasiertes 12mer zugegeben und das Oligonukleotidgemisch langsam (über etwa 3 Stunden) auf 35 °C abgekühlt, um Hybrisierung der Oligonukleotide zu ermöglichen. Hierauf wurden nach Zugabe von 5 mM DTT und 0,25 mM ATP die Oligonukleotide miteinander ligiert ("blunt-end"-Ligierung, siehe Teil A). Das Fehlen eines Phosphatrestes am 5'-Ende des 12mers verhinderte die Bildung von Dimeren; das überhängende Ende des 14mers ist nicht selbstkomplementär, es kann nicht zur Dimerisierung führen.

Ein Aliquot (25 pMole) des ligierten Oligonukleotidkomplexes wurde mit 80 Einheiten Sau3A verdaut, um das ins Interferongen passende Sau3A-Ende zu erzeugen. Hierauf wurde die Probe 10 Minuten auf 75 °C erhitzt, mit Phenol extrahiert und mit Äthanol gefällt. Hiermit war das Linkerfragment, das das Interferongen mit dem HindIII-geschnittenen pER103 erfindungsgemäß verbindet, fertiggestellt.

c. Verknüpfung von Interferongen und Oligonukleotid-Komples, Ligierung in pER103

Die isolierten Interferonfragmente (siehe Beispiel 2a), die etwa 1 pMol von Molekülen darstellen, wurden mit dem Sau3A-geschnittenen Oligonukleotidkomplex (etwa 25 pMol) durch Ligierung der kohäsiven Sau3A-Enden (siehe Teil A) verbunden. Wieder verhinderte das Fehlen eines Phosphatrestes am HindIII-Ende des Oligonukleotidkomplexes (12mer) den Aufbau von Multimeren. Es entstanden Interferongen-Moleküle, die auf beiden Seiten von einem Oligonukleotidkomplex mit einem freien HindIII-Ende flankiert waren. Nach Hitzedenaturierung der Ligase und Zugabe von 5 mM DTT und 0,25 mM ATP wurden diese Enden phosphoryliert (siehe Teil A), dann wurde eine Isopropanolfällung (siehe Teil A) durchgeführt, um die ebenfalls in der Ligasereaktion entstandenen Oligonukleotidkomplex-Dimeren abzutrennen. Hierauf wurde die Konstruktion mit 0,05 µg HindIII-geschnittenem, phosphatasebehandeltem pER103 ligiert (Ligierung von kohäsiven Enden, siehe Teil A). Die Phosphatasebehandlung (siehe Teil A) des HindIII-geschnittenen Plasmids verminderte dessen Rezirkularisierung in der Ligasereaktion.

Hiermit war ein Gemisch von Plasmiden fertiggestellt, das zu 50 % Interferonproduktionsplasmide enthielt (nämlich diejenigen Plasmide, die ein 34 pb Sau3A-AvaII-Fragment in der Ligierung mit dem 646 bp AvaII-Fragment am Genbeginn erhalten hatten - siehe Beispiel 2a).

d. Transformation von Escherichia coli HB 101 und Analyse der Transformation im Hinblick auf Interferonproduktion

Das gemäß Beispiel 2c hergestellte Plasmidgemisch wurde analog Beispiel le zur Transformation von Escherichia coli HB 101 verwendet (siehe Dworkin und Dawid, Dev. Biol. 76 , 435-448 (1980)). Etwa 20 % der erhaltenen Transformanten hatten Inserte (der Rest waren rezirkularisierte pER103-Plas-mide), von denen mehrere auf Interferonexpression untersucht wurden. Dazu wurden 100 ml Bakterienkultur in M9 Minimum Medium, dem alle Aminosäuren außer Trypthophan (20 µg/ml pro Aminosäure), sowie Thiamin (1 µg/ml), Glucose (0,2 %) und der Induktor des Tryptophanoperons Indol-(3)-acrylsäure (IAA, 20 µg/ml; siehe Hallewell und Emtage in Gene 9 , 27-47 (1980)), zugesetzt worden waren, zu einer optischen Dichte von 0,6-0,8 gezüchtet. Die Bakterien wurden durch Zentrifugation 10 Minuten bei 7000 U/min pelletiert, 1 x in 50 mM Tris-HCl, pH 8, 30 mM NaCl gewaschen und schließlich in 1,5 ml desselben Puffers suspendiert. Nach Inkubation mit 1 mg/ml Lysozym 30 Minuten lang auf Eis wurden die Bakterien 5 x gefroren und getaut und hierauf die Zellbruchstücke durch Zentrifugation eine Stunde bei 40 000 U/min entfernt. Der Überstand wurde steril filtriert und im Plaquereduktionstest mit V3-Zellen und Vesicular Stomatitis Virus auf Interferonaktivität getestet. Etwa die Hälfte aller Klone (mit Insert) zeigte beträchtliche Interferonexpression: 2 x $10^8$ Einheiten (internationale Referenzeinheiten) pro Liter Kultur.

e. Sequenzanalyse des Interferonproduktionsplasmids pER33

Einer dieser interferonproduzierenden Klone, welcher als pER33 bezeichnet wurde, wurde ausgewählt und von der Promotor/Operator-Region weg bis ins Interferongen hinein sequenziert, um die Richtigkeit des konstruierten Plasmides festzustellen. Die Sequenzanalyse wurde wieder nach Maxam und Gilbert (siehe Proc. Nat. Acad. Sci. USA 74 , 560-564 (1977)) durchgeführt und erfolgte von der am 3'-Ende radioaktiv markierten EcoRI-Spaltstelle ausgehend in Richtung Interferongen. Es ergab sich die erwartete Sequenz, ein Ausschnitt davon ist in Fig. 5 dargestellt. In diesem Ausschnitt sind alle zur Konstruktion von pER33 verwendeten Oligonukleotidfragmente zu sehen.

Die vorstehend erwähnten Eigenschaften belegen, daß das erfindunggemäß hergestellte Plasmid pER103 die Promotor/Operator-Sequenz des Tryptophanoperons von Serratia marcescens in Kombination mit einer synthetischen Ribosomenbindungssequenz enthält. Am Beispiel von einem α-Interferon konnte gezeigt werden, daß in das Plasmid pER103 in geeigneter Weise eingebaute Gene hohe Werte an Expression zeigen. Das Plasmid pER103 wurde in Escherichia coli K 12, HB 101 bei der Deutschen Sammlung von Mikroorganismen Grisebachstraße 8, D 3400 Göttingen, unter der DSM-Nr. 2773 am 27. Oktober 1983 gemäß Budapester Vertrag hinterlegt.

## Beispiel 3

## Konstruktion des Plasmids pER21/1

Die Konstruktion des Plasmids pER21/1 ist schematisch in Figur 6 dargestellt.

a) Präparation eines für ein α-Interferon codierendes Gen aus pER33

2 µg pER33 wurden mit den Restriktionsenzym EcoRI und BamHI geschnitten, wodurch zwei Fragmente der Längen ca. 1300 bp und ca. 4000 bp entstanden. Diese Fragmente wurden auf einem 1,2 % Agarosegel elektrophoretisch aufgetrennt. Das kürzere Fragment wurde durch Elektroelution aus dem Gel isoliert. Die Enden dieser DNA wurden durch Zugabe von je 1,25 nMol dATP, dGTP, dCTP und dTTP sowie von 2 Einheiten Klenowfragment der DNA-Polymerase I in stumpfe Enden überführt. Die DNA wurde durch Phenolextraktion und Präzipitation aus äthanolischer Lösung gereinigt und anschließend in 15 µl $H_2O$ aufgenommen.

Etwa 15 pMol EcoRI-Linker (New England Biolabs Inc.) wurden durch Zugabe von $\gamma$-$^{32}$p-ATP und T4-Polynukleotidkinase in 5 µl Reaktionslösung an den 5'Enden phosphoryliert. Nach Hitzeinaktivierung der Kinase wurde die DNA, 5 nMol ATP und 0,1 Einheiten T4-Ligase zugesetzt und 16 Stunden bei 14° C inkubiert. Das Reaktionsprodukt wurde durch Isopropanolfällung von niedermolekularen Substanzen gereinigt. Die DNA wurde abschließend mit 20 Einheiten Restriktionsenzym EcoRI geschnitten, nochmals durch Isopropanolfällung gereinigt und in 10 µl $H_2O$ gelöst.

b) Linearisierung des Plasmids pER33

Ca. 2 µg pER33 wurden mit dem Restriktionsenzym EcoRI behandelt. Anschließend wurde alkalische Phosphatase zugesetzt, um die 5'Phosphatreste zu entfernen. Die ca. 5300 bp lange, lineare DNA wurde durch Elektrophorese in einem 1,2 % Agarosegel und Elektroelution von restlichem, nicht geschnittenem pER33 gereinigt. Die DNA wurde mit Phenol und Äther extrahiert, aus äthanolischer Lösung gefällt und in 10 µl $H_2O$ gelöst.

c) Verknüpfung des linearisierten pER33 mit dem zusätzlichen Gen für ein α-Interferon

4 µl des mit EcoRI-Linkern versehenen DNA-Stückes, das das Gen plus regulatorische Elemente enthält, wurden mit 0,5 µl EcoRI-linearisiertem und dephosphoryliertem pER33 in 20 µl Reaktionslösung mit Hilfe von 0,1 Einheiten T4-Ligase miteinander ligiert. Nach 16 Stunden Inkubation bei 14° C wurde das Enzym durch Erhitzen auf 68° C zerstört.

d) Transformation von E.coli HB101 und Analyse der Transformation im Hinblick auf Interferonproduktion

E.coli HB101 wurde analog Beispiel le mit der DNA aus Beispiel 3c transformiert. Zwei der so hergestellten Klone wurden analog Beispiel 2d auf Interferonexpression mittels Plaquereduktionstests geprüft. Während der Klon pER33 bis zu 200 x $10^6$ Einheiten IFN-α pro 1 1 Kultur aufwies, ergaben sich mit einem der neuen Klone, der als pER21/1 bezeichnet wurde, überraschenderweise mehr als 300 x $10^6$ Einheiten IFN-α pro Liter Kultur.

e) Restriktionsenzymanalyse des pER21/1

Das Plasmid pER21/1 wurde in größerer Menge isoliert und mittels Restriktionsenzymverdauung mit HindIII analysiert. Da die in pER33 in die EcoRI Stelle eingebrachte DNA zwei identische EcoRI-Enden aufwies, waren zwei Orientierungen dieser DNA im pER21/1 möglich. Restriktionsenzymverdauung mit HindIII von pER21/1 mit parallel gerichteten Interferongenen sollte Fragmente der ungefähren Größe von 4100, 950 (2 Fragmente) und 450 bp zeigen. Wären die beiden Gene jedoch entgegengesetzt orientiert, sind Fragmente der ungefähren Größe von 4350, 950 (2 Fragmente) und 200 bp zu erwarten. Der Verdauung von ca. 2 µg pER21/1 mit dem Restriktionsenzym HindIII und anschließende Elektrophorese in einem 1,4 % Agarosegel ergab Fragmente der ungefähren Größe 4100, 950 und 450 bp. Daher sind die beiden Gene parallel zueinander orientiert (siehe Figur 6).

## Beispiel 4

### Konstruktion des Plasmids parpER33

Die Konstruktion des Plasmides parpER33 ist schmematisch in Fig. 7 dargestellt.

a) Präparation des par-Lokus

Etwa 200 pMol EcoRI Linker (New England Biolabs Inc.) wurden in 20 µl Reaktionslösung mit 9 Einheiten T4-Polynukleotidkinase und 10 nMol ATP an den Enden phorphoryliert, und das Enzym anschließend hitzeinaktiviert.

8 µg des Plasmids pPM31 wurden mit dem Restriktionsenzym AvaI geschnitten. Die Enden des linearisierten Plasmids wurden durch Zugabe von je 5 nMol dATP, dGTP, dTTP sowie 4 Einheiten des Enzyms Klenowfragment der Polymerase I in stumpfe Enden überführt. Die DNA wurde durch Phenolextraktion und Präzipitation aus äthanolischer Lösung gereinigt und in 40 µl $H_2O$ aufgenommen.

Die EcoRI Linker wurden zusammen mit der linearisierten und stumpfe Enden aufweisenden DNA in 70 µl Reaktionslösung mit 6 nMol ATP und 1 Einheit T4-Ligase 16 Stunden bei 14° C behandelt. Nach Hitzeinaktivierung des Enzyms wurde die Lösung auf 50 mM NaCl und 50 mM Tris-Cl pH = 7,6 eingestellt und die DNA mit 300 Einheiten EcoRI behandelt. Nach 2 Stunden Inkubation wurde das Restriktionsenzym hitzedenaturiert und die DNA elektrophoretisch in einem 1,4 % Agarosegel aufgetrennt. Das den par-Lokus beinhaltende Stück DNA von ca. 400 bp Länge wurde aus dem Gel elektroeluiert, durch Phenolextraktion und Fällung aus äthanolischer Lösung gereinigt und in 50 µl $H_2O$ gelöst. Dieses Stück DNA weist nun an seinen Enden EcoRI spezifische Überhänge auf.

b) Linearisierung des Plasmids pER33

Etwa 2 µg pER33 wurden mit dem Restriktionsenzym EcoRI behandelt. Anschließend wurden alkalische Phosphatase zugesetzt, um die 5'Phosphatreste zu entfernen. Die ca. 5300 bp lange, lineare DNS wurde durch Elektrophorese in einem 1,2 % Agarosegel und Elektroelution von restlichem, nicht geschnittenem pER33 gereinigt. Die DNA-Lösung wurde mit Phenol und Äther extrahiert, aus äthanolischer Lösung gefällt und in 50 µl $H_2O$ gelöst.

c) Verknüpfung des linearisierten pER33 mit der par-Lokus DNA

1 µl linearisierte pER33 DNA wurde mit 1 µl par-Lokus DNA in 20 µl Reaktionslösung mit Hilfe von

0,1 Einheiten T4-Ligase miteinander ligiert. Nach 16 Stunden Inkbuation bei 14°C wurde das Enzym hitzeinaktiviert.

d) Transformation von E.coli HB101 und Analyse der Plasmide aus den transformierten Bakterien E.coli HB101 wurde analog zu Beispiel le mit der DNA aus Beispiel 4c transformiert. Es wurden ca. 50 Kolonien erhalten. Aus zehn dieser Kolonien wurde eine geringe Menge Plasmid DNA isoliert (Birnboim und Doly, Nucleic Acid Research 7 , 1513-1523 (1979)) und mit dem Restriktionsenzym PstI geschnitten. Die Analyse durch Agarosegelektrophorese ergab, daß alle diese Plasmide den ca. 400 bp langen par-Lokus enthielten. Eines dieser Plasmide wurde ausgewählt und mit parpER33 bezeichnet.

e) Analyse des parpER33 im Hinblick auf Interferonproduktion und Stabilität

Analog Beispiel 2d wurden Bakterien, die entweder pER33 oder parpER33 enthielten, angezüchtet und anschließend im Plaquereduktionstest auf Interferongehalt geprüft. Beide Stämme zeigten etwa dasselbe Niveau der Interferonexpression.

In einem Langzeitversuch, der sich über 120 Bakteriengenerationen erstreckte, wurde die Stabilität der Plasmide pER33 und parpER33 in E.coli HB101 in Abwesenheit des Selektionsdruckes durch das Antibiotikum Ampicillin untersucht. In regelmäßigen Abständen wurden der Kultur Proben entnommen, und die Bakterien auf Interferongehalt geprüft. Es stellte sich heraus, daß die pER33 enthaltenden Bakterien nach ca. 60 Generationen die Interferonproduktion einstellten. Bakterien, die parpER33 enthielten, produzierten jedoch auch noch nach 120 Generationen unvermindert das durch den Klon 1F7 codierte reife α-Interferon.

Damit wurde gezeigt, daß das Einbringen des par-Lokus in pER33 ("parpER33") die Stabilität des Plasmids in E.coli HB101 erhöht.

## Verwendete Begriffe und Abkürzungen

| | |
|---|---|
| ATP | Adenosintriphosphat |
| Basenpaar: | 2 komplementäre Nukleotide, z.B. A-T, G-C |
| blunt end: | vollständig basengepaartes Ende eines DNA-Doppelstrangmoleküls, zum Unterschied von überhängenden Einzelstrang-Enden |
| bp: | Basenpaare |
| BSA: | Bovinserumalbumin |
| cDNA: | eine zu mRNA komplementäre DNA |
| codieren: | die Information für etwas tragen; DNA trägt in der Nukleotidsequenz die Information für die Aminosäuresequenz eines Proteins |
| Codon: | Gruppe von 3 Nukleotiden, die für eine bestimmte Aminosäure oder aber für den Abbruch der Polypeptidsynthese codiert |
| dephosphorylieren: | eine Phosphatgruppe entfernen |
| DNA: | Deoxyribonukleinsäure |
| DTT: | Dithiothreitol |
| Elektrophorese: | Trennung von (DNA-) Molekülen im elektrischen Feld |
| Expression: | Umsetzung der Information eines Gens in mRNA durch Transkription und in weiterer Folge in Polypeptid durch Translation |
| Gen: | Abschnitt auf der DNA, der die Information für ein bestimmtes Transkipt (RNA-Molekül) trägt, das in weiterer Folge in ein Protein übersetzt werden kann |
| Genprodukt: | RNA (Transkript), Protein (Translationsprodukt) |
| Hybridisierung (von Nukleinsäuren): | Ausbildung von stabilen Komplexen zwischen zueinander komplementären DNA-Strängen |
| Hybridplasmid: | Plasmid, das einen DNA-Abschnitt fremden Ursprungs enthält |
| Initiationscodon: | das Codon ATG, das für Methionin codiert und den Start der Translation signalisieren kann |
| Insert: | das Stück fremder DNA, das sich in einem Hybridplasmid befindet |

| | |
|---|---|
| Kinase: | Enzym, das 5' OH-Gruppen an DNA- und RNA-Molekülen phosphorylieren kann |
| kinasieren: | mit 5'-Phosphatgruppen versehen |
| Klon: | Bakterienkolonie, von einem einzelnen Bakterium abstammend |
| kohäsive Enden: | überhängende Einzelstrangenden eines DNA-Moleküls, die miteinander hybridisieren können |
| komplementär: | zueinander passend (Nukleotide in der DNA: A ist komplementär zu T, G ist komplementär zu C) |
| Ligase: | Enzym, das verschiedene DNA-Moleküle kovalent miteinander verbinden kann |
| ligieren: | kovalent miteinander verbinden (DNA-Moleküle) |
| mRNA: | messenger RNA, ist eine für ein Polypeptid codierende RNA |
| Nukleotid: | Baustein einer DNA oder RNA (A= Adenosin, C = Cytidin, G = Guanosin, T = Thymidin, U = Uracil) |
| Nukleotidsequenz: | Abfolge der Nukleotide in einem DNA- oder RNA-Molekül |
| Oligonukleotid: | wenige miteinander durch Phosphodiesterbindungen verknüpfte Nukleotide (kurzer einzelsträngiger DNA-Abschnitt) |
| Operator: | Teil der regulatorischen Region eines Operons, an den der Repressor bindet, wodurch Transkription verhindert wird |
| Operon: | Gruppe von (bakteriellen) Genen, die von einer Operator-Region aus reguliert werden |
| Phosphatase: | Enzym, das 5'-Phosphatgruppen von DNA-Molekülen entfernen kann |
| Promotor: | DNA-Sequenz, die zur Bindung des Enzyms RNA-Polymerase befähigt ist |
| RBS: | siehe Ribosomenbindungssequenz |
| Restriktionsendonuklease (Restriktionsenzym): | Enzym, das bei einer bestimmten symmetrischen DNA-Sequenz den DNA-Doppelstrang spalten kann |
| Restriktionsfragmente: | Bruchstücke einer DNA, die durch Verdauung mit einer Restriktionsendonuklease entstehen |
| Ribosomenbindungssequenz: | Teil der mRNA, der ans Ribosom binden kann |
| RNA: | Ribonukleinsäure |
| RNA-Polymerase: | Enzym, das einen zu DNA komplementären RNA-Strang synthetisieren kann |
| Sequenz: | siehe Nukleotidsequenz |
| Terminationscodon: | Codon, das das Ende der Translation signalisiert |
| Transformant: | Bakterium, das fremde DNA (ein Plasmid) durch Transformation erhalten hat |
| Transformation: | Einschleusen fremder (Plasmid-) DNA in Bakterien |
| Transkription: | Synthese von RNA komplementär zu einer DNA-Matrize |
| Translation: | Umsetzung der Information von mRNA in ein Polypeptid |
| Tris: | Trishydroxymethylaminoethan |

Kurze Beschreibung der Illustrationen

Figur 1

zeigt eine schematische Darstellung der Konstruktion des Plasmids pER103. Die Fragmentgrößen sind nicht maßstabgerecht dargestellt.

Figur 2

zeigt die HaeIII-Verdauungsmuster der Plasmide pBR322 und pER103. Das 192 bp-Fragment von pBR322 ist in pER103 durch ein Fragment von ca. 270 bp ersetzt.

Figur 3

zeigt die Nukleotidsequenz von pER103 zwischen der EcoRI und der HindIII-Spaltstelle. Der Rest des Plasmides entspricht dem großen HindIII-EcoRI-Fragment von pBR322.

Figur 4

ist eine schematische Darstellung der Konstruktion des Plasmids pER33. ↓ :PstI-Spaltstelle, ♀ : Sau3A-Spaltstelle, ↑ : AvaII-Spaltstelle. Mit Ausnahme der Restriktionskarte des 1F7-Inserts sind die Fragmente nicht maßstabgerecht dargestellt.

Figur 5

zeigt einen Ausschnitt eines Sequenzgeles, auf dem die Verbindung Promotor-RBS-Interferongen zu sehen ist. Alle in der Konstruktion von pER33 verwendeten Oligonukleotidfragmente sind vorhanden. Zum besseren Verständnis ist dieselbe Sequenz darunter noch einmal doppelsträngig angegeben; die einzelnen Oligonukleotidbausteine sind eingezeichnet.

Figur 6

ist eine schematische Darstellung der Konstruktion des Plasmids pER21/1. Die Fragmente bzw. Plasmide sind nicht maßstabsgetreu abgebildet.

Figur 7

ist eine schematische Darstellung der Konstruktion des Plasmids parpER33. Die Fragmente und Plasmide sind nicht maßstabgetreu wiedergegeben.

## Ansprüche

1. Eine DNA-Sequenz der Formel

```
5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
                  |<---------Promotor----------------------

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
---Promotor/Operator--------------------->|<--RBS--------->|
                                                    HindIII
```

2. Eine DNA-Sequenz gemäß Anspruch 1, dadurch gekennzeichnet, daß daran anschließend an die Ribosomenbindungssequenz eine Linkersequenz und die Sequenz eines Strukturgens für ein beliebiges Polypeptid in derartiger Form angefügt ist,' daß dieses durch Bakterien zur Expression gebracht wird und die Kombination der DNA-Sequenzen derartig erfolgt, daß die DNA-Sequenz des Strukturgens an die DNA-Linkersequenz durch 3',5'-Phosphodiesterbindung gekoppelt ist.

3. Eine DNA-Sequenz gemäß Anspruch 2, dadurch gekennzeichnet, daß das Strukturgen für ein Interferon codiert.

4. Eine DNA-Sequenz gemäß Anspruch 2, dadurch gekennzeichnet, daß das Strukturgen mit der im PstI-Insert des Klons 1F7 (DMS-Nr. 2362) enthaltenen DNA-Sequenz, welche nur für ein reifes Interferon codiert, identisch ist.

5. Eine DNA-Sequenz gemäß Anspruch 2, 3 oder 4, dadurch gekennzeichnet, daß diese die Formel

```
5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
                    |<-----------Promotor--------------------

GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
---Promotor/Operator----------------->|<--RBS-------->|
                                                HindIII

          |<--im PstI Insert des Klons 1F7 (DSM-Nr. 2362) enthal-
          tene für ein reifes IFN-α codierende DNA-Sequenz--->
TAAAGATGTGTGATCTGCCTCAAA
ATTTCTACACACTAGACGGAGTTT
|<---Linker---->|
```

aufweist.

6. Eine DNA-Ribosomenbindungs/Linkersequenz gemäß Anspruch 2 der Formel

```
5'  TAAGGAGGTTTAAGCTTAAAGATGTGT
3'  ATTCCTCCAAATTCGAATTTCTACACACTAG
                                Sau3A
```

7. Eine DNA-Linkersequenz gemäß Anspruch 2 der Formel

```
5'  AGCTTAAAGATGTGT
3'      ATTTCTACACACTAG
```

**8.** Eine DNA-Sequenz, welche aus der Linkersequenz des Anspruchs 7 und der im PstI-Insert des Klons 1F7 (DSM-Nr. 2362) vorhandenen für ein reifes IFN-α codierenden DNA-Sequenz ohne das $NH_2$-terminale Cystein dieses Interferons besteht, wobei beide Teile über die Sau3A-Enden so ligiert sind, daß das gesamte reife IFN-α codiert wird.

**9.** Ein in Bakterien replizierbares Plasmid, enthaltend eine oder mehrere DNA-Sequenzen gemäß den Ansprüchen 4 bis 8.

**10.** Das Plasmid pER103, erhältlich aus dem E. coli Stamm hinterlegt unter der DSM-Nr. 2773 bei der Deutschen Sammlung von Mikroorganismen, Grisebachstraße 8, D-3400 Göttingen.

**11.** Ein Plasmid gemäß Anspruch 9, dadurch gekennzeichnet, daß dieses zusätzlich einen par-Lokus enthält.

**12.** Ein Plasmid gemäß Anspruch 9 oder 11, dadurch gekennzeichnet, daß das Plasmid pBR322 anstelle des 29 bp langen EcoRI/HindIII-Fragmentes eine DNA-Sequenz der Formel

```
5'AATTCACGCTGATCGCTAAAACATTGTGCAAAAAGAGGGTTGACTTTGCCTTC
3'    GTGCGACTAGCGATTTTGTAACACGTTTTTCTCCCAACTGAAACGGAAG
                |<----------Promotor-------------------

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
---Promotor/Operator------------------>|<--RBS-------->|
                                                 HindIII
```

enthält, an welche anschließend an die Hin dIII-Spaltstelle eine Linkersequenz der Formel

```
              |<--IFN-Gen-------->
AGCTTAAAGATGTGT
   ATTTCTACACACTAG
|<-----Linker------>|
              Sau3A
```

und eine für ein Interferon codierende Sequenz eingefügt ist.

**13.** Das Plasmid pER33 wie in Beispiel 2 beschrieben und enthaltend die DNA-Sequenz gemäß Anspruch 5.

**14.** Das Plasmid pER21/1 wie in Beispiel 3 beschrieben.

**15.** Das Plasmid parpER33 wie in Beispiel 4 beschrieben.

**16.** Verfahren zur Herstellung einer DNA-Sequenz gemäß Anspruch 1, dadurch gekennzeichnet, daß eine 90 bp lange Promotor/Operator-Sequenz des Tryptophanoperons von Serratia marcescens mit einer Ribosomenbindungssequenz der Formel

```
5'   TAAGGAGGTTTA
3'   ATTCCTCCAAATTCGA
```

ligiert wird.

**17.** Verfahren zur Herstellung einer DNA-Sequenz gemäß den Ansprüchen 2 bis 5, dadurch gekennzeichnet, daß eine DNA-Sequenz gemäß Anspruch 1 mit einer DNA-Sequenz der Formel

```
                  |<--Gen-->
AGCTTAAAGATGTGT
     ATTTCTACACACTAG
|<-----Linker------>|
```

verbunden wird.

**18.** Verfahren gemäß Anspruch 17, dadurch gekennzeichnet, daß als Strukturgen ein Gen für Interferon, vorzugsweise das Strukturgen gemäß Anspruch 4, verwendet wird.

**19.** Verfahren zur Herstellung einer DNA-Sequenz gemäß Anspruch 8, dadurch gekennzeichnet, daß man das 646 bp lange AvaII-Fragment des PstI-Inserts des Klons 1F7 mit einem 34 bp langen Fragment, das man durch Behandlung des 177 bp langen Sau3A-Fragmentes des PstI-Inserts des Klons 1F7 mit AvaII erhält, ligiert und das so erhaltene Fragment mit einem Oligonukleotid der Formel

```
5'   AGCTTAAAGATGTGT
3'        ATTTCTACACACTAG
```

ligiert.

**20.** Verfahren zur Herstellung eines Plasmids gemäß Anspruch 9, dadurch gekennzeichnet, daß man ein Plasmid mit einer DNA-Sequenz gemäß den Ansprüchen 4 bis 8 kombiniert.

**21.** Verfahren zur Herstellung des Plasmids pER103 gemäß Anspruch 10, dadurch gekennzeichnet, daß man in das Plasmid pBR322 durch Restriktionsendonukleasespaltung das plasmideigene 29 bp lange EcoRI-Hin dIII Fragment entfernt und an dessen Stelle eine DNA-Sequenz gemäß Anspruch 1 durch Ligasereaktion einfügt.

**22.** Verfahren zur Herstellung eines Plasmids gemäß Anspruch 11, dadurch gekennzeichnet, daß eine in einem bakteriellen Plasmid enthaltene DNA-Sequenz gemäß den Ansprüchen 4 bis 8 und gegebenenfalls ein aus pPM31 isolierter par-Lokus eingefügt wird.

**23.** Verfahren zur Herstellung des Plasmids pER33 gemäß Anspruch 13, dadurch gekennzeichnet, daß man in die HindIII-Spaltstelle des Plasmids pER103 gemäß Anspruch 10 die DNA-Sequenz gemaß Anspruch 5 mittels Ligasereaktion einfügt.

**24.** Verfahren zur Herstellung des Plasmids pER21/1 gemäß Anspruch 14, dadurch gekennzeichnet, daß man in das Plasmid pER33 gemäß Anspruch 13, welches mit EcoRI linearisiert ist, ein DNA-Fragment mit der Länge von ca. 1300 bp, welches durch Behandlung des Plasmids pER33 mit den Restriktionsenzymen EcoRI und BamHI erhalten wird, über einen EcoRI/BamHI-Linker einfügt.

**25.** Verfahren zur Herstellung des Plasmids parpER33 gemäß Anspruch 15, dadurch gekennzeichnet, daß

man in das Plasmid pER33 gemäß Anspruch 13, welches durch das Restriktionsenzym EcoRI linearisiert ist, den aus dem Plasmid pPM31 isolierten par-Lokus einfügt.

26. Mikroorganismus, dadurch gekennzeichnet, daß ein Bakterium mit einem Plasmid gemäß den Ansprüchen 9 bis 15 transformiert ist.

27. Verfahren zur Herstellung eines Mikroorganismus gemäß Anspruch 26, dadurch gekennzeichnet, daß ein Plasmid gemäß den Ansprüchen 9 und 11 bis 15 mit einem bakteriellen Wirt transformiert wird.

28. Verfahren zur Herstellung eines Polypeptids, dadurch gekennzeichnet, daß ein bakterieller Wirt mit einem Plasmid gemäß den Ansprüchen 9 bis 15 zur regulierten Produktion des Polypeptids transformiert wird und das so exprimierte Polypeptid isoliert wird.

29. Ein reifes Polypeptid mit IFN-α Aktivität, dadurch gekennzeichnet, daß es durch Züchtung eines Bakteriums, das ein eine DNA-Sequenz gemäß den Ansprüchen 4, 5 oder 8 enthaltendes Plasmid enthält und durch anschließende Isolierung gewonnen werden kann.

## Claims

1. A DNA sequence of formula

```
5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
                  |<-----------Promoter--------------------

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
--Promoter/Operator ------------------>|<--RBS ------>|
                                                HindIII.
```

2. A DNA sequence according to claim 1, characterized in that, in the ribosome binding sequence, there is a linker sequence and the sequence of a structural gene for any desired polypeptide is added in a form such that this polypeptide is expressed by bacteria and the combination of the DNA sequences is such that the DNA sequence of the structural gene is coupled to the DNA linker sequence by 3',5'-phosphodiester binding.

3. A DNA sequence according to claim 2, characterized in that the structural gene codes for an interferon.

4. A DNA sequence according to claim 2, characterized in that the structural gene is identical to the DNA sequence contained in the PstI insert of clone 1F7 (DMS No. 2362) which codes only for a mature interferon.

5. A DNA sequence according to claim 2, 3 or 4, characterized in that it has the formula

```
5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
                    |<---------Promoter ----------------

GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
--Promoter/Operator ------------------>|<--RBS -------->
                                                HindIII
```

← DNA sequence coding for a mature IFN-α and contained in the PstI insert of the clone 1F7 (DSM No. 2362) →

```
TAAAGATGTGTGATCTGCCTCAAA
ATTTCTACACACTAGACGGAGTTT
|<---Linker--->|.
```

6. A DNA ribosome binding/linker sequence according to claim 2 of the formula

```
5'  TAAGGAGGTTTAAGCTTAAAGATGTGT
3'  ATTCCTCCAAATTCGAATTTCTACACACTAG
                                Sau3A .
```

7. A DNA linker sequence according to claim 2 of the formula

```
5'  AGCTTAAAGATGTGT
3'      ATTTCTACACACTAG  .
```

8. A DNA sequence which consists of the linker sequence of claim 7 and the DNA sequence coding for a mature IFN-α and present in the PstI insert of clone 1F7 (DSM No. 2362), without the $NH_2$-terminal cysteine of this interferon, the two parts being ligated via the Sau3A ends in such a way that the entire mature IFN-α is coded.

9. A plasmid which is replicable in bacteria, containing one or more DNA sequences according to claims 4 to 8.

10. The plasmid pER103, obtainable from the E. coli strain deposited under DSM No. 2773 at the Deutschen Sammlung von Mikroorganismen, Grisebachstraße 8, D-3400 Göttingen.

11. A plasmid according to claim 9, characterized in that it additionally contains a par-locus.

12. A plasmid according to claim 9 or 11, characterized in that the plasmid pBR322 contains, instead of the 29 bp long EcoRI/HindIII fragment, a DNA sequence of the formula

```
5'AATTCACGCTGATCGCTAAAACATTGTGCAAAAAGAGGGTTGACTTTGCCTTC
3'    GTGCGACTAGCGATTTTGTAACACGTTTTTCTCCCAACTGAAACGGAAG
                   |<------------Promoter--------------

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
---Promoter/Operator------------------>|<---RBS-------->|
                                                  HindIII
```

which is followed, after the HindIII cleavage site, by a linker sequence of the formula

```
                 |<--IFN-gene---->
      AGCTTAAAGATGTGT
          ATTTCTACACACTAG
      |<- - -Linker-- -->|
                 Sau3A
```

and a sequence coding for an interferon.

13. The plasmid pER33 as described in Example 2 and containing the DNA sequence according to claim 5.

14. The plasmid pER21/1 as described in Example 3.

15. The plasmid parpER33 as described in Example 4.

16. Process for preparing a DNA sequence according to claim 1, characterized in that a 90 bp long promoter/operator sequence of the tryptophan operon of Serratia marcescens is ligated with a ribosome binding sequence of the formula

```
5'  TAAGGAGGTTTA
3'  ATTCCTCCAAATTCGA.
```

17. Process for preparing a DNA sequence according to claims 2 to 5, characterised in that a DNA sequence according to claim 1 is bound to a DNA sequence of the formula

```
             |<---Gene--->
   AGCTTAAAGATGTGT
       ATTTCTACACACTAG
   |<- - -Linker- - ->| .
```

**18.** Process according to claim 17, characterized in that a gene for interferon, preferably the structural gene according to claim 4, is used as the structural gene.

**19.** Process for preparing a DNA sequence according to claim 8, characterized in that the 646 bp long AvaII fragment of the PstI insert of clone 1F7 is ligated with a 34 bp long fragment obtained by treating the 177 bp long Sau3A fragment of the PstI insert of clone 1F7 with AvaII, and the resulting fragment is ligated with an oligonucleotide of the formula

```
5'  AGCTTAAAGATGTGT
3'       ATTTCTACACACTAG.
```

**20.** Process for preparing a plasmid according to claim 9, characterized in that a plasmid is combined with a DNA sequence according to claims 4 to 8.

**21.** Process for preparing the plasmid pER103 according to claim 10, characterized in that the 29 bp long EcoRI-HindIII fragment native to the plasmid is removed from the plasmid pBR322 by restriction endonuclease cleaving and in its place a DNA sequence according to claim 1 is inserted by ligase reaction.

**22.** Process for preparing a plasmid according to claim 11, characterized in that a DNA sequence contained in a bacterial plasmid according to claims 4 to 8 and optionally a par-locus isolated from pPM31 is inserted.

**23.** Process for preparing the plasmid pER33 according to claim 13, characterized in that the DNA sequence according to claim 5 is inserted into the HindIII cleavage site of the plasmid pER103 according to claim 10 by ligase reaction.

**24.** Process for preparing the plasmid pER21/1 according to claim 14, characterized in that a DNA fragment with a length of about 1300 bp, obtained by treating the plasmid pER33 with the restriction enzymes EcoRI and BamHI, is inserted in the plasmid pER33 according to claim 13, linearised with EcoRI, by means of an EcoRI/BamHI linker.

**25.** Process for preparing the plasmid parpER33 according to claim 15, characterized in that the par-locus isolated from the plasmid pPM31 is inserted into the plasmid pER33 according to claim 13, which has been linearised by means of the restriction enzyme EcoRI.

**26.** Microorganism, characterised in that a bacterium is transformed with a plasmid according to claims 9 to 15.

**27.** Process for preparing a microorganism according to claim 26, characterized in that a plasmid according to claims 9 and 11 to 15 is transformed with a bacterial host.

**28.** Process for preparing a polypeptide, characterized in that a bacterial host is transformed with a plazmid according to claims 9 to 15 for the regulated production of the polypeptide and the polypeptide thus expressed is isolated.

**29.** A mature polypeptide with IFN-α activity, characterized in that it can be obtained by growing a bacterium which contains a plasmid containing a DNA sequence according to claims 4, 5 or 8 and by subsequent isolation.

## Revendications

**1.** Sêquence d'ADN de formule

5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
|←———— Promoteur -————————

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
—— Promoteur/opérateur ————————→|← Séquence de liaison des ribosomes

HindIII

2. Séquence d'ADN selon la revendication 1, caractérisée en ce qu'il y est ajouté, à la suite de la séquence de liaison aux ribosomes, une séquence linker et la séquence d'un gène de structure pour un polypeptide quelconque, sous une forme telle que celui-ci est amené à être exprimé par des bactéries et en ce que la combinaison des séquences d'ADN a lieu de façon telle que la séquence d'ADN du gène de structure est couplée à la séquence linker d'ADN par liaison 3',5'-phosphodiester.

3. Séquence d'ADN selon la revendication 2, caractérisée en ce que le gène de structure code pour un interféron.

4. Séquence d'ADN selon la revendication 2, caractérisée en ce que le gène de structure est identique à la séquence d'ADN contenue dans l'insert PstI du clone 1F7 (n° DSM 2362), laquelle séquence ne code que pour un interféron mature.

5. Séquence d'ADN selon la revendication 2, 3 ou 4, caractérisée en ce qu'elle présente la formule

5'AATTCACGCTGATCGCTAAAACATTGTGCAAAAAGAGGGTTGACTTTGCCTTC
3'      GTGCGACTAGCGATTTTGTAACACGTTTTTCTCCCAACTGAAACGGAAG
|←———— Promoteur ————

GCGAACCAGTTAACTAGTACACAAGTTCACGGCAACGGTAAGGAGGTTTAAGCT
CGCTTGGTCAATTGATCATGTGTTCAAGTGCCGTTGCCATTCCTCCAAATTCGA
—— Promoteur/opérateur ————→|←Séquence de liaison aux ribosomes

HindIII

|←-- Séquence d'ADN codant pour un IFN mature, contenue dans l'insert PstI du clone 1F7 (n° DSM 2362)--→

TAAAGATGTGTGATCTGCCTCAAA
ATTTCTACACACTAGACGGAGTTT
|←---Linker----→|

**6.** Séquence de liaison aux ribosomes/séquence linker d'ADN selon la revendication 2, de formule

5'  TAAGGAGGTTTAAGCTTAAAGATGTGT
3'  ATTCCTCCAAATTCGAATTTCTACACACTAG
Sau3A

**7.** Séquence linker d'ADN selon la revendication 2, de formule

5'  AGCTTAAAGATGTGT
3'      ATTTCTACACACTAG

**8.** Séquence d'ADN qui est constituée de la séquence linker de la revendication 7 et de la séquence d'ADN codant pour un IFN ∝ mature présente dans l'insert PstI du clone 1F7 (n° DSM 2362), sans la cystéine NH2-terminale de cet interféron, les deux parties étant ligaturées par l'intermédiaire des extrémités, Sau3A de sorte que l'ensemble de l'IFN ∝ mature complet soit codé.

**9.** Plasmide réplicable dans des bactéries, contenant une ou plusieurs séquences d'ADN selon les revendications 4 à 8,

**10.** Plasmide pER103 qui peut être obtenu à partir de la souche de E. coli déposée sous le n° DSM 2773 à la Collection Allemande de Microorganismes (Deutsche Sammlung von Mikroorganismen), Grisebachstrasse 8, D-3400 Göttingen.

**11.** Plasmide selon la revendication 9, caractérisé en ce qu'il contient en outre un locus par.

**12.** Plasmide selon la revendication 9 ou 11, caractérisé en ce que le plasmide pBR322 contient, au lieu du fragment EcoRI/HindIII de 29 pb de long, une séquence d'ADN de formule

```
5'AATTCACGCTGATCGCTAAAACATTGTGCAAAAAGAGGGTTGACTTTGCCTTC
3'    GTGCGACTAGCGATTTTGTAACACGTTTTTCTCCCAACTGAAACGGAAG
               |<---------- Promoteur -----------

GCGAACCAGTTAACTAGTACACAAGTTCACGGCAACGGTAAGGAGGTTTA
CGCTTGGTCAATTGATCATGTGTTCAAGTGCCGTTGCCATTCCTCCAAATTCGA
--- Promoteur/opérateur ----------------->|<-- Séquence de liaison
                                               aux ribosomes
                                               HindIII
```

à laquelle est ajoutée, à la suite du site de clivage Hind III, une séquence linker de formule

```
                 |<--Gène d'IFN
AGCTTAAAGATGTGT
    ATTTCTACACACTAG
|<-----Linker------>|
                Sau3A
```

et une séquence codant pour un interféron.

**13.** Plasmide pER33 comme décrit dans l'exemple 2 et contenant la séquence d'ADN selon la revendication 5.

**14.** Plasmide pER21/1 comme décrit dans l'exemple 3.

**15.** Plasmide parpER33 comme décrit dans l'exemple 4.

**16.** Procédé de préparation d'une séquence d'ADN selon la revendication 1, caractérisé en ce qu'une séquence promoteur/opérateur de 90 pb de long de l'opéron du tryptophane de Serratia marcescens est ligaturée avec une séquence de liaison aux ribosomes de formule

```
5'  TAAGGAGGTTTA
3'  ATTCCTCCAAATTCGA
```

**17.** Procédé de préparation d'une séquence d'ADN selon les revendications 2 à 5, caractérisé en ce qu'une séquence d'ADN selon la revendication 1 est liée à une séquence d'ADN de formule

|←Gène

AGCTTAAAGATGTGT

ATTTCTACACACTAG

|←-----Linker------→|

18. Procédé selon la revendication 17, caractérisé en ce qu'on utilise comme gène de structure un gène pour de l'interféron, de préférence le gène de structure selon la revendication 4.

19. Procédé de préparation d'une séquence d'ADN selon la revendication 8, caractérisé en ce qu'on ligature le fragment AvaII de 646 bp de long de l'insert PstI du clone 1F7 avec un fragment de 34 pb de long que l'on obtient par traitement du fragment Sau3A de 177 pb de long de l'insert PstI du clone 1F7 au moyen d'AvaII et en ce qu'on ligature le fragment ainsi obtenu avec un oligonucléotide de formule

5' AGCTTAAAGATGTGT

3' ATTTCTACACACTAG

20. Procédé de préparation d'un plasmide selon la revendication 9, caractérisé en ce qu'on combine un plasmide avec une séquence d'ADN selon les revendications 4 à 8.

21. Procédé de préparation du plasmide pER103 selon la revendication 10, caractérisé en ce que dans le plasmide pBR322 on élimine le fragment EcoRI-HindIII de 29 pb de long, propre au plasmide, par clivage par une endonucléase de restriction et en que qu'à sa place on introduit une séquence d'ADN selon la revendication 1, par réaction d'une ligase.

22. Procédé de préparation d'un plasmide selon la revendication 11, caractérisé en ce qu'on introduit une séquence d'ADN selon les revendications 4 à 8, contenue dans un plasmide bactérien et éventuellement un locus par isolé de pPM31.

23. Procédé de préparation du plasmide pER33 selon la revendication 13, caractérisé en ce qu'on introduit, dans le site de clivage HindIII du plasmide pER103 selon la revendication 10, la séquence d'ADN selon la revendication 5, au moyen d'une réaction de ligase.

24. Procédé de préparation du plasmide pER21/1 selon la revendication 14, caractérisé en ce qu'on introduit dans le plasmide pER33 selon la revendication 13, lequel est linéarisé au moyen de EcoRI, un fragment d'ADN ayant une longueur d'environ 1300 pb, lequel est obtenu par traitement du plasmide pER33 par les enzymes de restriction EcoRI et BamHI, par l'intermédiaire d'un linker EcoRI/BamHI.

25. Procédé de préparation du plasmide parpER33 selon la revendication 15, caractérisé en ce qu'on introduit le locus par isolé du plasmide pPM31 dans le plasmide pER33 selon la revendication 13, lequel est linéarisé par l'enzyme de restriction EcoRI.

26. Microorganisme, caractérisé en ce qu'une bactérie est transformée par un plasmide selon les revendications 9 à 15.

27. Procédé de préparation d'un microorganisme selon la revendication 26, caractérisé en ce qu'un plasmide selon les revendications 9 et 11 à 15 est transformé par un hôte bactérien.

28. Procédé de préparation d'un polypeptide, caractérisé en ce qu'un hôte bactérien est transformé par un plasmide selon les revendications 9 à 15 pour la production régulée du polypeptide et en ce que le polypeptide ainsi exprimé est isolé.

**29.** Polypeptide mature présentant une activité INF ∝, caractérisé en ce qu'il peut être obtenu par culture d'une bactérie qui contient un plasmide contenant une séquence d'ADN selon les revendications 4, 5 ou 8 et par isolement subséquent.

Figur 1

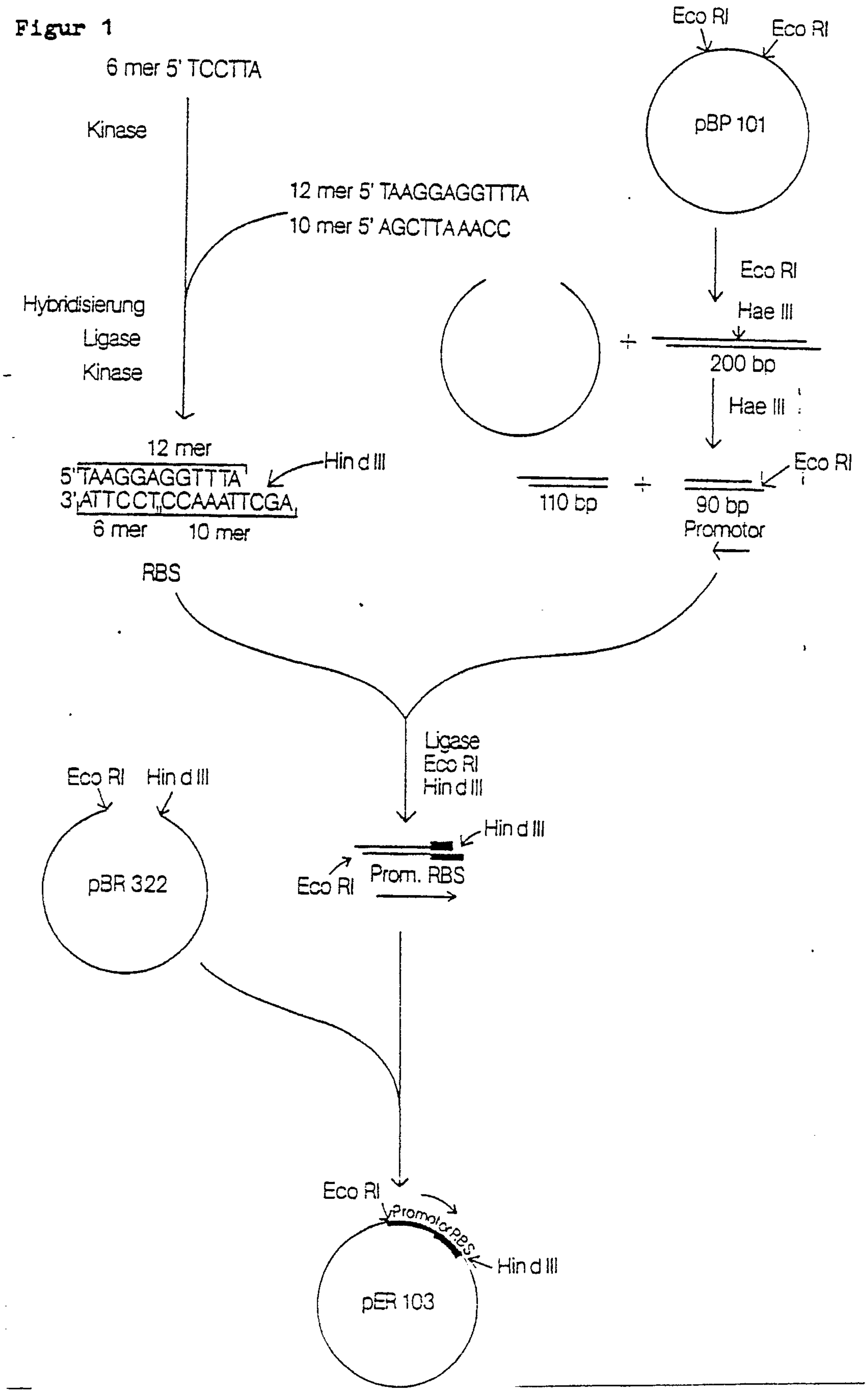
6 mer 5' TCCTTA
Kinase
12 mer 5' TAAGGAGGTTTA
10 mer 5' AGCTTAAACC
Hybridisierung
Ligase
Kinase
12 mer
5' TAAGGAGGTTTA
3' ATTCCTCCAAATTCGA
6 mer
10 mer
Hin d III
RBS
Eco RI
Eco RI
pBP 101
Eco RI
Hae III
200 bp
Hae III
110 bp
90 bp
Promotor
Eco RI
Ligase
Eco RI
Hin d III
Eco RI
Hin d III
pBR 322
Hin d III
Eco RI
Prom. RBS
Eco RI
Promotor RBS
Hin d III
pER 103

Figur 2

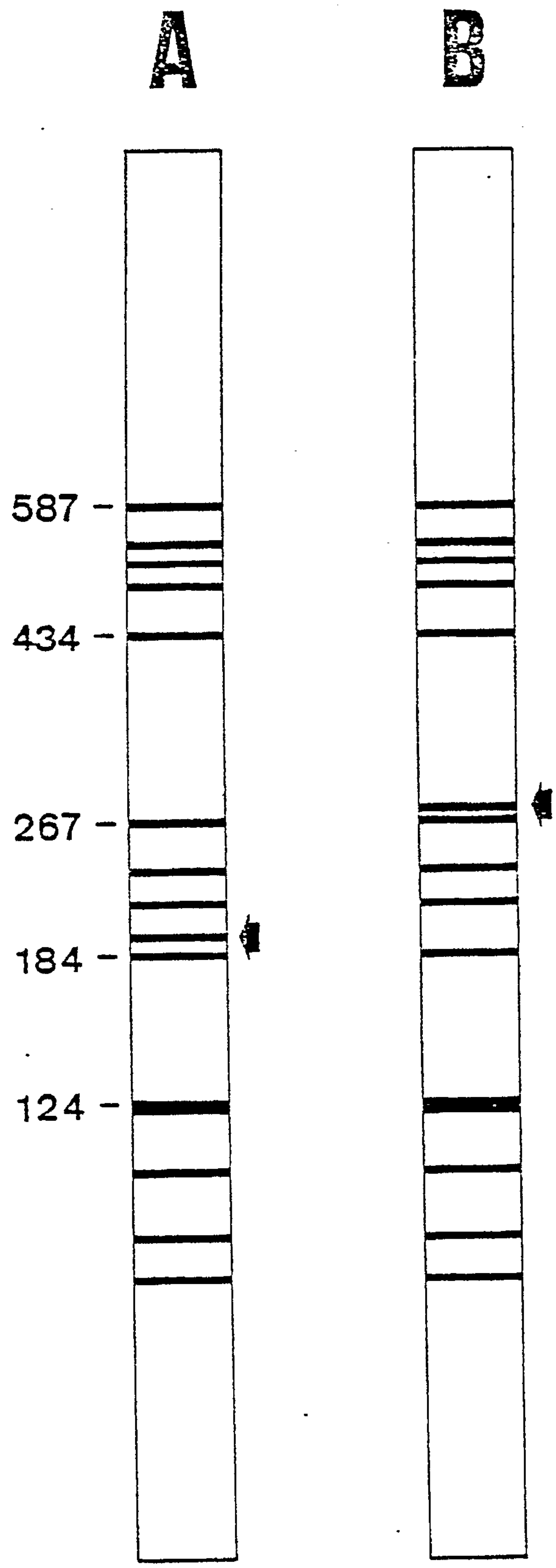

**Figur 3**

**Nukleotidsequenz des 103 bp-Eco RI-Hin dIII-Fragmentes von pER 103:**

```
  Eco RI
5'AATTCACGCTGATCGCTAAAACATTGTGCAAAAAGAGGGTTGACTTTGCCTTC
3'     GTGCGACTAGCGATTTTGTAACACGTTTTTCTCCCAACTGAAACGGAAG
                        >--------------Promotor ----------

 GCGAACCAGTTAACTAGTACACAAGTTCACGGCAACGGTAAGGAGGTTTA
 CGCTTGGTCAATTGATCATGTGTTCAAGTGCCGTTGCCATTCCTCCAAATTCGA
                                                 Hin dIII
 ---- Promotor/Operator ---------------->|<---RBS ------>|
```

Figur 4

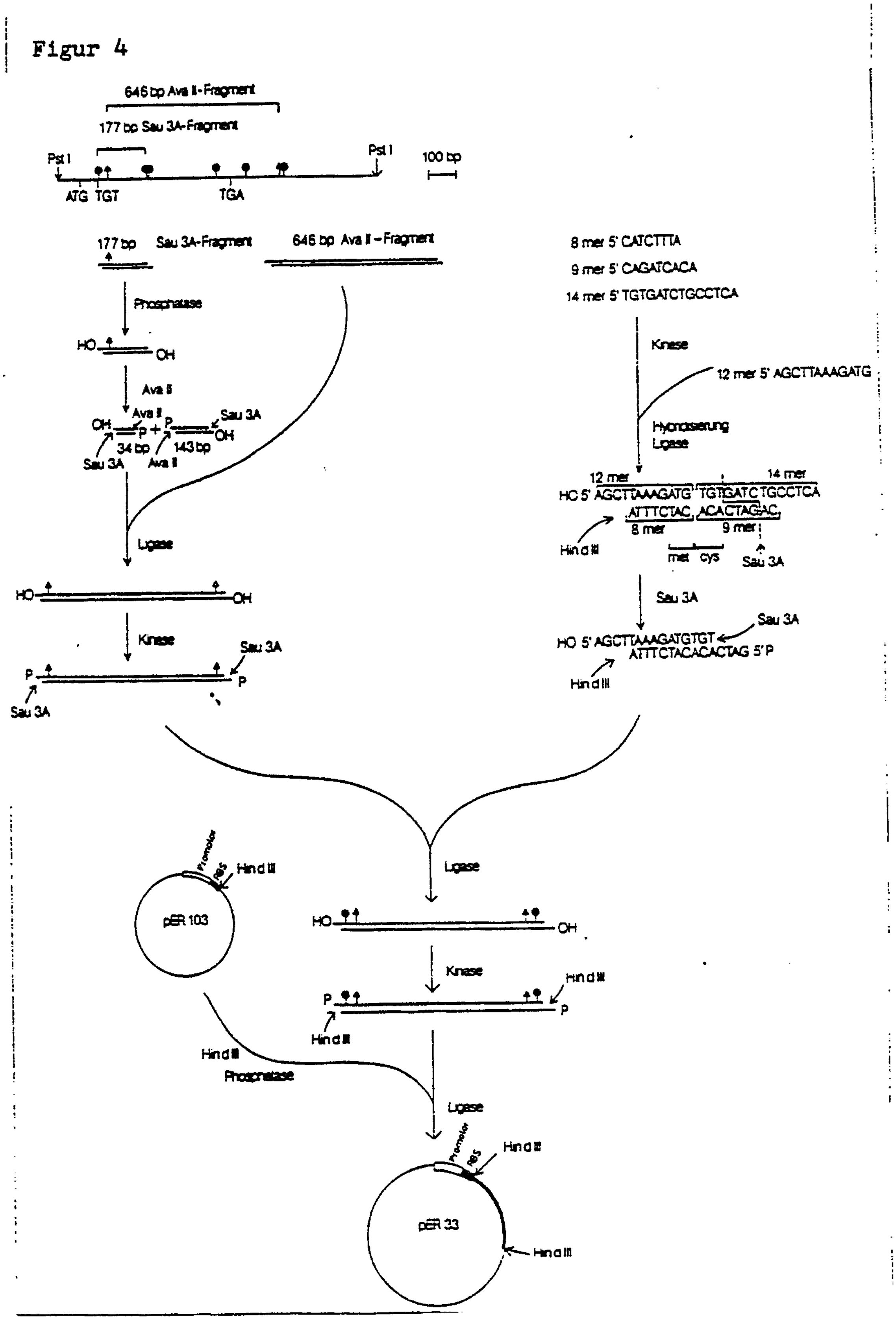
646 bp Ava II-Fragment
177 bp Sau 3A-Fragment
Pst I
Pst I
100 bp
ATG TGT
TGA
177 bp Sau 3A-Fragment
646 bp Ava II-Fragment
8 mer 5' CATCTTTA
9 mer 5' CAGATCACA
14 mer 5' TGTGATCTGCCTCA
Phosphatase
Kinase
12 mer 5' AGCTTAAAGATG
Ava II
Hybridisierung
Ligase
Sau 3A
34 bp
143 bp
12 mer
14 mer
HO 5' AGCTTAAAGATG TGTGATCTGCCTCA
ATTTCTAC ACACTAGAC
8 mer
9 mer
Hind III
met cys
Ligase
Sau 3A
HO 5' AGCTTAAAGATGTGT
ATTTCTACACACTAG 5' P
Hind III
Kinase
pER 103
Promotor
RBS
Phosphatase
pER 33

Figur 5

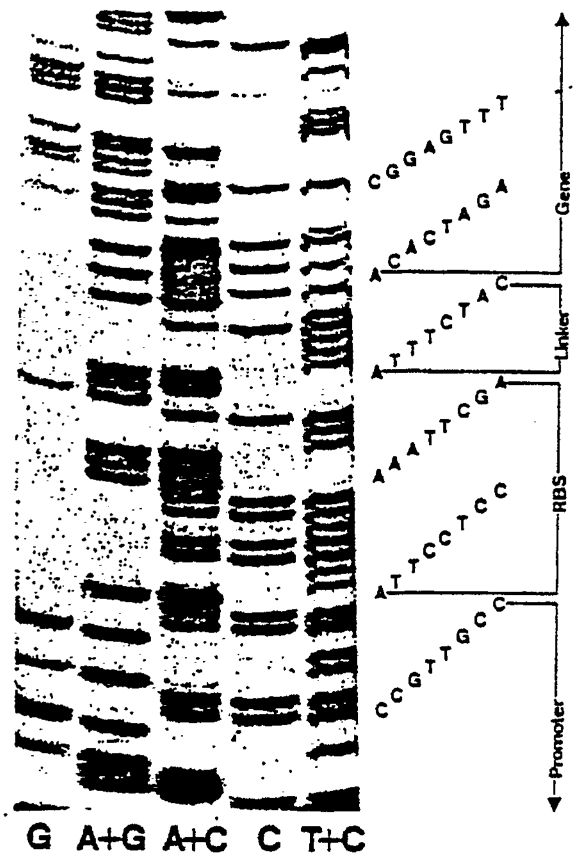


Eco RI

5'AATTCACGCTGATCGCTAAAACATTGTGCAAAAAGAGGGTTGAC
3'     GTGCGACTAGCGATTTTGTAACACGTTTTTCTCCCAACTG

Promotor

TTTGCCTTCGCGAACCAGTTAACTAGTACACAAGTTCACGGCAA
AAACGGAAGCGCTTGGTCAATTGATCATGTGTTCAAGTGCCGTT

Promotor/Operator

CGGTAAGGAGGTTTAAGCTTAAAGATGTGTGATCTGCCTCAAA...
GCCATTCCTCCAAATTCGAATTTCTACACACTAGACGGAGTTT...

RBS   Linker   Gen

Figur 6

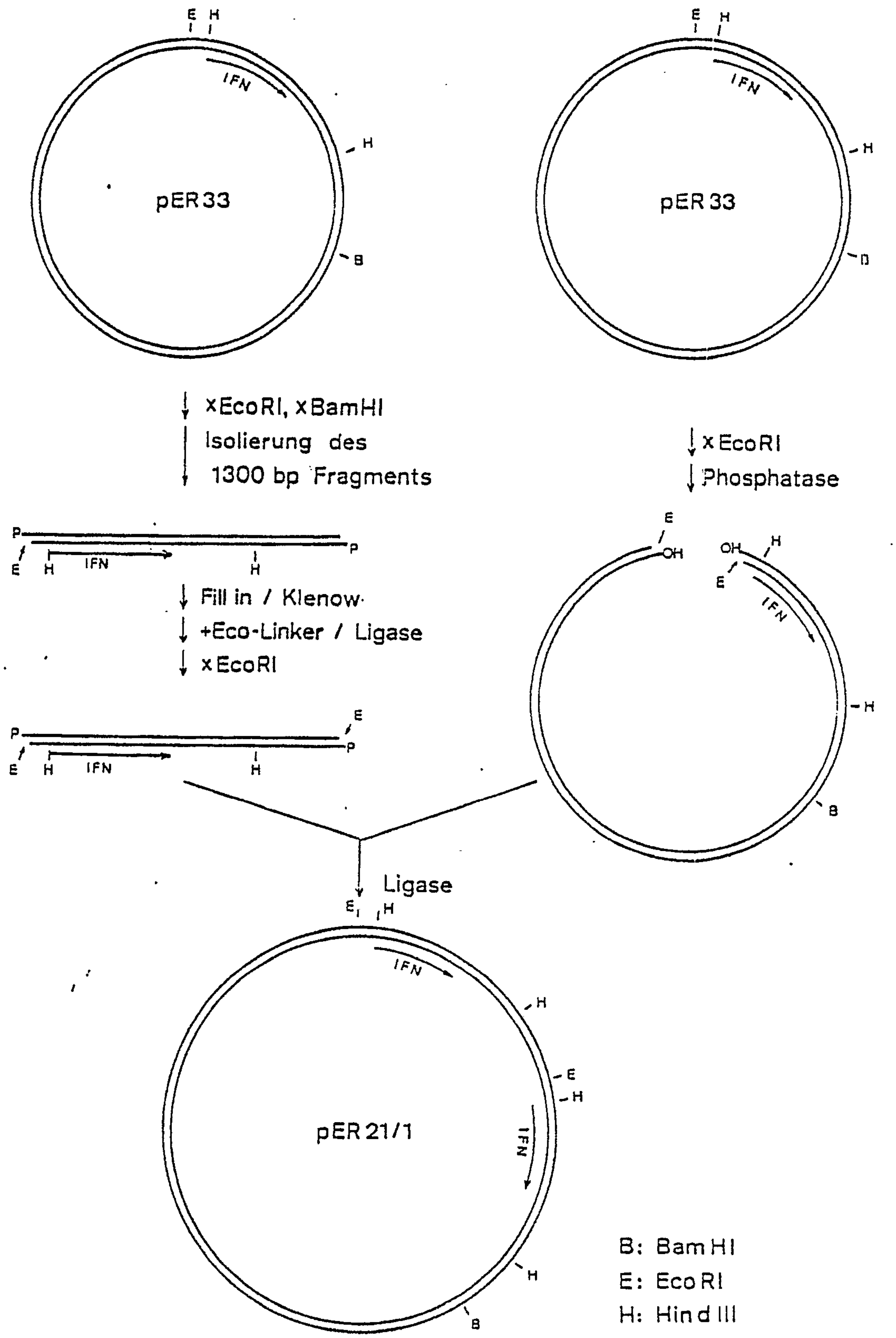
E
H
IFN
H
pER33
B
E
H
IFN
H
pER 33
B
xEcoRI, xBamHI
Isolierung des
1300 bp Fragments
xEcoRI
Phosphatase
P
P
E
H
IFN
H
Fill in / Klenow
+Eco-Linker / Ligase
xEcoRI
P
E
P
E
H
IFN
H
E
OH
OH
H
E
IFN
H
B
Ligase
E
H
IFN
H
E
H
pER 21/1
IFN
H
B
B: BamHI
E: EcoRI
H: Hind III

Figur 7

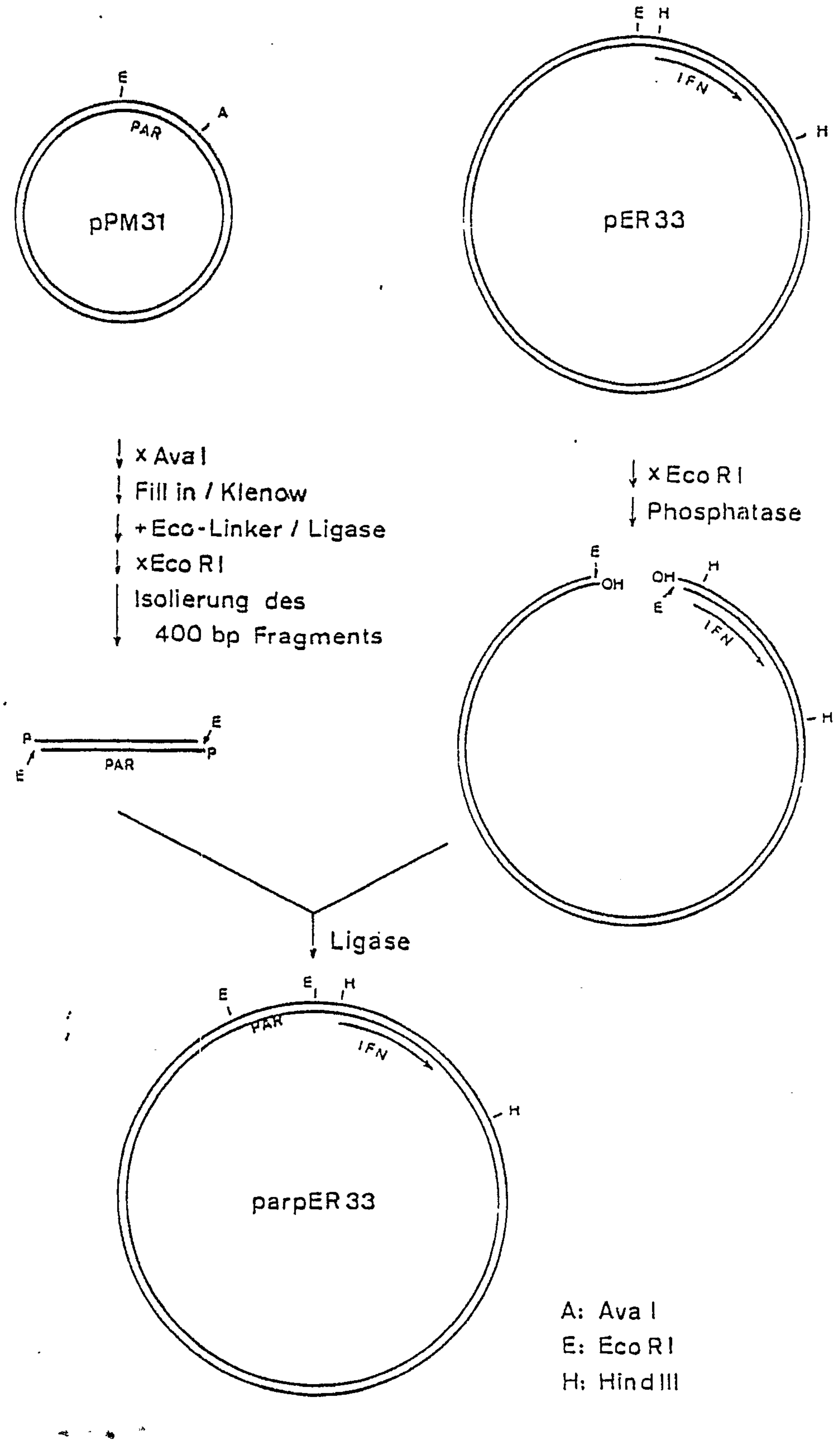
E
PAR
A
pPM31
E H
IFN
H
pER33
x Ava I
Fill in / Klenow
+Eco-Linker / Ligase
xEco R I
Isolierung des
400 bp Fragments
x Eco R I
Phosphatase
E
OH
OH
H
E
IFN
H
P
E
P
E
PAR
Ligase
E
E
H
PAR
IFN
H
parpER33
A: Ava I
E: Eco R I
H: Hind III